(19) Europäisches Patentamt
European Patent Office
Office européen des brevets

(11) EP 1 877 091 B1

(12) # EUROPEAN PATENT SPECIFICATION

(45) Date of publication and mention of the grant of the patent:
**25.03.2015 Bulletin 2015/13**

(21) Application number: **06741437.5**

(22) Date of filing: **26.04.2006**

(51) Int Cl.:
***A61K 45/06*** (2006.01) ***A61K 31/4196*** (2006.01)
***A61K 31/135*** (2006.01) ***A61K 31/513*** (2006.01)
***A61K 31/5375*** (2006.01) ***A61K 31/661*** (2006.01)
***A61P 31/18*** (2006.01)

(86) International application number:
**PCT/CA2006/000685**

(87) International publication number:
**WO 2006/114001 (02.11.2006 Gazette 2006/44)**

(54) **METHOD FOR IMPROVING PHARMACOKINETICS OF PROTEASE INHIBITORS AND PROTEASE INHIBITOR PRECURSORS**

VERFAHREN ZUR VERBESSERUNG DER PHARMAKOKINETIK VON PROTEASEINHIBITOREN UND PROTEASEINHIBITORVORLÄUFERN

PROCEDE D'AMELIORATION DE LA PHARMACOCINETIQUE D'INHIBITEURS DE PROTEASE ET DE LEURS PRECURSEURS

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **27.04.2005 US 675082 P**

(43) Date of publication of application:
**16.01.2008 Bulletin 2008/03**

(73) Proprietor: **TaiMed Biologics, Inc.**
**Taipei City 115 (TW)**

(72) Inventors:
- **WU, Jinzi, J.**
  **Dollard-des-Ormeaux, Québec H9A 3K3 (CA)**
- **STRANIX, Brent, Richard**
  **Pointe-Claire, Québec H9R 3L7 (CA)**
- **GE, Michael**
  **St-Laurent, Québec H4M 2M2 (CA)**
- **MILOT, Guy**
  **Verdun QC H3E 0A2 (CA)**
- **PETRELLA, Marco**
  **Montréal, Québec H1P 3J3 (CA)**
- **PANCHAL, Chandra**
  **London, Ontario N6P 1E3 (CA)**

(74) Representative: **Harris, Jennifer Lucy et al**
**Kilburn & Strode LLP**
**20 Red Lion Street**
**London WC1R 4PJ (GB)**

(56) References cited:
**WO-A1-97/27180** **WO-A1-02/064551**
**WO-A1-2004/054586** **WO-A1-2005/066131**
**US-B1- 6 632 816** **US-B1- 6 632 816**
**US-B2- 6 703 403**

- **STRANIX BRENT R ET AL: "Lysine sulfonamides as novel HIV-protease inhibitors: Nepsilon-disubstituted ureas" BIOORGANIC & MEDICINAL CHEMISTRY LETTERS, vol. 14, no. 15, 2 August 2004 (2004-08-02), pages 3971-3974, XP002544355 ISSN: 0960-894X**
- **KEAYS ET AL: "HIV infections" 20040201, vol. 5, no. 2, 1 February 2004 (2004-02-01), pages 54-57, XP005770445**
- **POIRIER J M ET AL: "Interet et limites du suivi therapeutique des inhibiteurs de la protease du VIH" REVUE FRANÇAISE DES LABORATOIRES, EDITIONS SCIENTIFIQUES ET MEDICALES ELSEVIER, vol. 2004, no. 365, 1 September 2004 (2004-09-01), pages 67-72, XP004825188 ISSN: 0338-9898**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **WAI LANG CHU: "HIV protease inhibitor impresses in preclinical trials"[Online] 18 October 2005 (2005-10-18), XP002544356 Retrieved from the Internet: URL:http: //www.drugresearcher.com/content/ view/print/ 24442>**
- **FITZSIMMONS M.E. ET AL.: 'Selective biotransformation of the human immunodefiency virus protease inhibitor Saquinavir by human small-intestinal Cytochrome P4503A4' DRUG METAB. DISP. vol. 25, no. 2, 1997, pages 256 - 266, XP008125985**
- **TRELUYER J.M. ET AL.: 'Oxidative metabolism of Amprenavir in the human liver. Effect of the CYP3A Maturation' DRUG METAB. DISP. vol. 31, no. 3, 2003, pages 275 - 281, XP008125986**
- **ANDRADE A. ET AL.: 'HIV-Related Drug Metabolism and Cytochrome P450 Enzymes' ADIS CLIN. CARE vol. 12, no. 11, 2000, pages 91 - 95, XP001539545**
- **KEMPF D.J. ET AL.: 'Pharmacokinetic Enhancement of Inhibitors of the Human Immunodeficiency Virus Protease by Coadministration with Ritonavir' ANTIMICR. AGENTS CHEM. vol. 41, no. 3, March 1997, pages 654 - 660, XP002480911**

## Description

### TECHNICAL FIELD OF THE INVENTION

This invention relates to compounds and compositions for use in improving the pharmacokinetics of protease inhibitors and protease inhibitor precursors and related pharmaceutical compositions. More particularly, the present invention relates to compounds and compositions for use in improving the pharmacokinetics of protease inhibitors and protease inhibitor precursors by co-administering ritonavir.

### BACKGROUND OF THE INVENTION

Inhibitors of the HIV viral protease have been developed relatively recently and their use began only in 1996. Currently, they are considered the most effective drugs against HIV infection. Unfortunately, most current proteases inhibitors are relatively large hydrophobic molecules that possess rather low bioavailability. A high pill burden is therefore required to attain the therapeutic dose in a patient. This is a deterrent, which too often results in patient non-compliance and inadequate treatment results. This situation leads to sub-optimal therapeutic drug concentration that in turns leads to the development of HIV resistant strains. Consequently, there is an urgent need to improve the solubility and bioavailability of proteases inhibitors.

Examples of improved compounds have been developed in the form of prodrugs of aspartyl protease inhibitors such as described, for example, in U.S. patent no. 6,436,989 to Hale et al. This patent shows a novel class of molecules characterized by favourable aqueous solubility, high oral bioavailability and facile *in vivo* generation of the active ingredient. However, it is well known that HIV has the ability to develop resistance to the currently available drugs. Thus, there is a need for alternative HIV protease inhibitors active towards wild-type and resistant viral strains. Thus, molecules derived from current HIV protease inhibitors showing enhanced solubility and bioavailability is desirable to fight resistant viral strains.

A unique class of aromatic derivatives which are inhibitors of aspartyl proteases is described in U.S. patent no. 6,632,816 to Stranix et al..

This patent includes, more particularly, *N*-synthetic amino acid substituted L-lysine derivatives possessing potent aspartyl protease inhibitory properties. However, it would be advantageous to improve these derivatives by enhancing aqueous solubility and bioavailability in order to reduce the pill burden and to favour patient's compliance. Since it is challenging to generate active protease inhibitors, specifically toward wild-type and resistant strains, the formation of derivatives of original HIV protease inhibitors such as inhibitors described in U.S. patent no. 6,632,816 to Stranix et al*,* known to be active toward resistant strains represents a viable route with considerable advantages. More particularly, generation of compounds and formulations with enhanced aqueous solubility, oral bioavailability, time of duration and formulation properties along with other advantages is desirable in the development of an effective drug. Protease inhibitors with improved pharmacokinetics are therefore desirable.

### SUMMARY OF THE INVENTION

Lysine-based compounds with increased solubility and improved oral bioavailability are described herein and have been described in United States patent application No. 10/902,935 filed on August 2, 2004 and published on February 2, 2006 under No. 2006/0025592A1. These compounds may readily be cleaved *in vivo* to release an active ingredient which has an affinity for aspartyl proteases and which may act as a protease inhibitor. More particularly, the active ingredient may bind, for example, to an HIV aspartyl protease (U.S. patent no. 6,632,816) and may inhibit this enzyme. The Lysine-based compounds are also referred herein as a protease inhibitor precursor.

Upon *in vivo* physiological conditions (e.g., metabolic, enteric and/or gastrointestinal conditions, etc.) the Lysine-based compounds, allow for the release of a protease inhibitor (e.g., aspartyl protease inhibitor). The Lysine-based compounds may thus serve as means for improving the solubility and/or bioavailability of protease inhibitors and therefore may reduce the pill burden and/or reduce dosages needed for inhibition. Improved treatment of HIV-infected patients and favourable patient's compliance may consequently occur.

The Lysine-based compounds described herein may be used alone or in combination with other therapeutic or prophylactic agents for the treatment or prophylaxis of HIV infection.

The active ingredient when released from the Lysine-based compound may act, for example, on aspartyl protease of HIV-1 including mutated and non-mutated HIV-1 viral strain (e.g., NL4.3) as well as on protease of HIV-2 (mutated or non-mutated) or even on protease of related viruses (e.g. retroviruses (e.g. SIV, etc.) etc.).

Other means to increase the pharmacokinetics of Lysine-based compounds described herein or of the active ingredients disclosed in U.S. patent no. 6,632,816 to Stranix et al*,* are investigated herein.

In accordance with the present invention, there is provided a pharmaceutical composition comprising:

a lysine-based compound of formula II

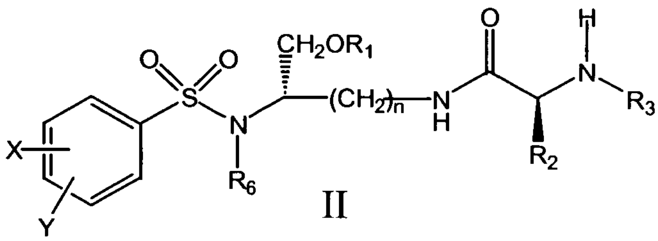

II

or a pharmaceutically acceptable salt thereof, wherein:

n is 4,
X is 4-$NH_2$,
YisH
$R_6$ is *iso*-butyl,
$R_3$ is $CH_3O$-CO-,
X' and Y' are H,
$R_2$ is a diphenylmethyl group of formula IV

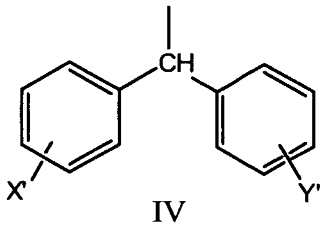

IV

,

$R_1$ is $(HO)_2P(O)$;
ritonavir; and

a pharmaceutically acceptable carrier;

wherein the ratio (w/w) of said lysine-based compound, or pharmaceutically acceptable salt thereof, to ritonavir is 6:1 to 3:1. The pharmaceutical composition is capable of improving the pharmacokinetics of a Lysine-based compound.

The present invention also provides a pharmaceutical combination comprising:

a lysine-based compound of formula II

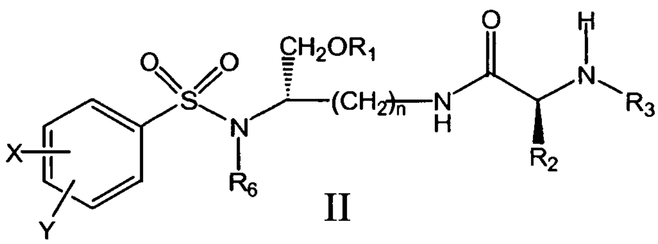

II

or a pharmaceutically acceptable salt thereof, wherein:

n is 4,
X is 4-$NH_2$,
YisH
$R_6$ is *iso*-butyl,
$R_3$ is $CH_3O$-CO-,
X' and Y' are H,

$R_2$ is a diphenylmethyl group of formula IV

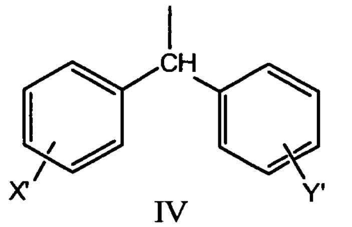

IV

,

R, is $(HO)_2P(O)$; and

ritonavir;

wherein the ratio (w/w) of said lysine-based compound, or pharmaceutically acceptable salt thereof, to ritonavir is 6:1 to 3:1.

The ritonavir and the compound of formula III, or a pharmaceutically acceptable salt thereof, may be administered either separately, simultaneously or sequentially. The ritonavir and the compound of formula II, or a pharmaceutically acceptable salt thereof may be administered at different time intervals.

For example, when administering a combination of a compound of formula III (or a pharmaceutically acceptable salt thereof) and ritonavir the two therapeutic agents may be formulated as separate composition which may be administered separately at the same time or at different times, using the same route of administration or different routes of administration, at the same administration site or at different administration sites etc. Alternatively, the therapeutic agents may be administered as a single composition either orally, by injection, etc.

The pharmaceutical composition of the invention or the pharmaceutical combination of the invention as described herein may be used for the treatment or prevention of a retroviral infection (e.g., HTLV, HIV, i.e., HIV-1, HIV-2) or for the treatment or prevention of acquired immunodeficiency syndrome (AIDS).

In an additional aspect, the present invention relates to the use of the lysine-based compound of formula II, wherein n, X, Y, X', Y', $R_1$, $R_2$, $R_3$ and $R_6$ are as defined herein or a pharmaceutically acceptable salt thereof, and ritonavir in the manufacture of a drug (or pharmaceutical composition) for the treatment or prevention of an HIV infection (for reducing the risk or probability of HIV infection), or again for reducing HIV burden in a mammal in need thereof or for the treatment or prevention of acquired immunodeficiency syndrome (AIDS) (for reducing the risk or probability of developing AIDS) for delaying the apparition of AIDS (symptoms) or for reducing AIDS symptoms in a mammal in need thereof, wherein the ratio (w/w) of the lysine-based compound and the ritonavir is 6:1 to 3:1.

A "mammal in need" is to be understood herein, without limitation, as an individual infected with HIV (i.e., at any stage of HIV infection, e.g., primary infection, symptomatic, asymptomatic, AIDS) or at risk of having an HIV infection. A "mammal in need" therefore, may comprise, in addition to individuals who has an acute HIV infection, a chronic HIV infection or AIDS, individuals (e.g., health care worker, an individual who had unprotected sexual intercourse, policemen, etc.) who may have, for example, come into contact with a possible source of contamination with HIV.

The present invention, additionally provides the lysine based compound of formula II

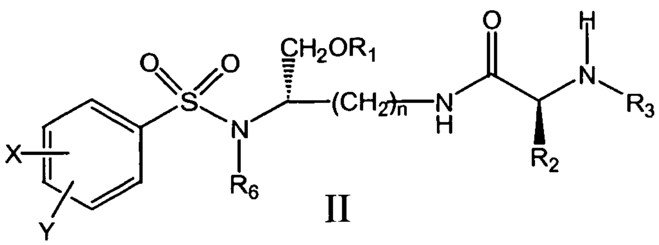

II

wherein n, X, Y, X', Y', $R_1$, $R_2$, $R_3$, and $R_6$ are as defined in claim 1 or a pharmaceutically acceptable salt thereof, and ritonavir for use in treating or preventing an HIV infection or treating or preventing AIDS wherein ritonavir is in an amount which is sufficient to reduce the metabolism of the lysine based compound of formula II and wherein the ratio (w/w) of said lysine based compound to ritonavir is 6:1 to 3:1.

Also in accordance with the present invention, the pharmaceutical composition may be administered, for example, orally.

Further in accordance with the present invention, the pharmaceutical composition may be administered, for

example, twice daily.

The Lysine-based compound of formula II which may be used to carry out the present invention may include also be represented by a compound of formula IIa;

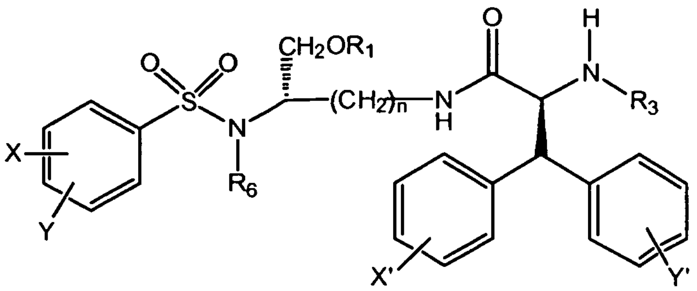

IIa

pharmaceutically acceptable salts thereof (e.g., for example, when the compound of the present invention comprises an amino group, the pharmaceutically acceptable salt may be an ammonium salt), wherein $R_1$, $R_3$, $R_4$, $R_6$, n, X, Y, X', Y' are as defined herein.

More particularly, the present invention provides a pharmaceutical composition which may comprise;

a) a compound of formula IIa

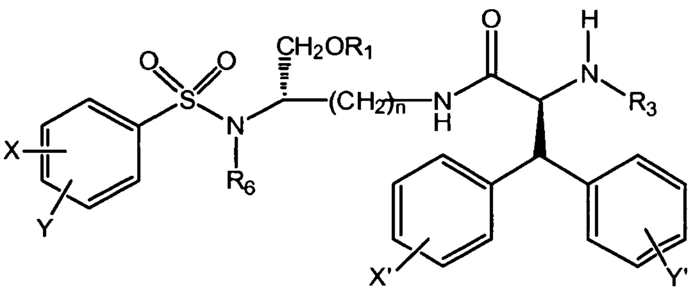

IIa

and pharmaceutically acceptable salts thereof,
wherein X, Y, X', Y', n, $R_1$, $R_3$ and $R_6$ are as defined herein;

b) ritonavir, and;

c) a pharmaceutically acceptable carrier, wherein the ratio (w/w) of said lysine-based compound, or a pharmaceutically acceptable salt thereof, to ritonavir is 6:1 to 3:1.

Any compound which is a precursor of an active ingredient described herein may be used to carry out the present invention and is also encompassed by the present invention.

Further in accordance with the present invention, administration of the compound of formula II and the ritonavir may be performed, for example, separately, simultaneously or sequentially.

In accordance with a particular embodiment of the present invention, administration of the compound of formula II and the ritonavir may be performed, for example, by administering a) a first pharmaceutical composition which may comprise a compound of formula II and a pharmaceutically acceptable carrier and b) a second pharmaceutical composition which may comprise ritonavir and a pharmaceutically acceptable carrier.

Further in accordance with the present invention, administration of the compound of formula II and ritonavir may be performed, for example, by administering a single pharmaceutical composition which may comprise a compound of formula II, ritonavir and a pharmaceutically acceptable carrier.

In accordance with an embodiment of the present invention, the HIV may be an HIV-1.

Also in accordance with an embodiment of the present invention, the HIV may be one that has a reduced

susceptibility to Ritonavir.

Further in accordance with an embodiment of the present invention, the HIV-1 may be one that possesses an aspartyl protease having one or more mutations (a mutation conferring a resistance to one or more of protease inhibitor mentioned herein).

The compounds listed herein are exemplary embodiments of compounds which may be used to carry out the present invention and it is to be understood that the present invention is not restricted to these compounds only.

The term "pharmaceutically effective amount" refers to an amount effective in treating, preventing or reducing the risk or probability of HIV infection or of reducing HIV burden. The term "pharmaceutically effective amount" also refers to an amount effective in treating, preventing or reducing the risk or probability of developing acquired immunodeficiency syndrome (AIDS), for delaying the apparition of AIDS, or reducing AIDS symptoms. It is also to be understood herein that a "pharmaceutically effective amount" may be construed as an amount giving a desired therapeutic effect, either taken into a single or multiple doses or in any dosage or route or taken alone or in combination with other therapeutic agents. In the case of the present invention, a "pharmaceutically effective amount" may be understood as an amount having an inhibitory effect (partial or complete) on HIV (HIV-1 and HIV-2 as well as related viruses (e.g., HTLV-I and HTLV-II, and simian immunodeficiency virus (SIV))) infection cycle (e.g., inhibition of replication, reinfection, maturation, budding etc.) and on any organism which rely on aspartyl proteases for its life cycle. An inhibitory effect is to be understood herein as an effect such as a reduction in the capacity of an organism (e.g. HIV) to reproduce itself (replicate), to re-infect surrounding cells, etc, or even a complete inhibition (or elimination) of an organism.

The terms "HIV protease" and "HIV aspartyl protease" are used interchangeably and includes, for example, the aspartyl protease encoded by the human immunodeficiency virus type 1 or 2.

The term "prophylactically effective amount" refers to an amount effective in preventing or reducing the risk or probability of HIV infection in a patient. As used herein, the term "patient" or "individual" refers to a mammal, including for example, a human.

The terms "pharmaceutically acceptable carrier", "pharmaceutically acceptable adjuvant" and "physiologically acceptable vehicle" refer to a non-toxic carrier or adjuvant that may be administered to a patient, together with one or more compounds of the present invention, and which does not destroy the pharmacological activity thereof.

The term "precursor" refers to a compound, such as a Lysine-based compound which is able to be converted into an active ingredient *in vitro* or *in vivo.* For example, the compound PL- 461 is a precursor of compound PL-100 as when administered to an individual, PL-461 is converted into PL-100 *in vivo* (e.g., under physiological conditions).

The term "derivative" refers to a compound which has been chemically synthesized from an original compound. For example, when considering the chemical synthesis of PL-461, PL- 461 is a derivative of PL-100.

The term "consisting essentially of means that the pharmaceutical composition includes the specified materials and may include other material that does not materially affect the basic characteristics of the pharmaceutical composition.

Pharmaceutically acceptable derivatives of the compounds of formula II and where applicable pharmaceutically acceptable salts thereof such as, for example, ammonium salts are described herein. A "pharmaceutically acceptable derivative" means any pharmaceutically acceptable salt, ester, or salt of such ester, of a compound of this invention or any other compound which, upon administration to a recipient (a mammal), is capable of providing (directly or indirectly) a an active compound or an antivirally active metabolite or residue thereof.

It is to be understood herein that a "straight alkyl group of 1 to 6 carbon atoms" includes for example, methyl, ethyl, propyl, butyl, pentyl, hexyl.

It is to be understood herein that a "branched alkyl group of 3 to 6 carbon atoms" includes for example, without limitation, *iso*-butyl, *tert*-butyl, 2-pentyl, 3-pentyl, etc.

It is to be understood herein, that a "cycloalkyl group having 3 to 6 carbon" includes for example, without limitation, cyclopropyl, cyclobutyl, cyclopentyl, cyclocyclohexyl (i.e., $C_6H_{11}$).

Salts derived from appropriate bases include alkali metal (e.g., sodium), alkaline earth metal (e.g., magnesium), ammonium and N - $(C_{1-4}alkyl)_4^+$ salts.

The compounds described herein contain one or more asymmetric carbon atoms and thus may occur as racemates and racemic mixtures, single enantiomer, diastereomeric mixtures and individual diastereoisomers. All such isomeric forms of these compounds are expressly included in the present invention. Each stereogenic carbon may be of the R or S configuration.

Pharmaceutically acceptable salts of the compounds described herein include those derived from pharmaceutically acceptable inorganic and organic acids and bases. Examples of such acid salts include: acetate, adipate, alginate, aspartate, benzoate, benzenesulfonate, bisulfate, butyrate, citrate, camphorate, camphorsulfonate, cyclopentanepropionate, digluconate, dodecylhydrogensulfate, dodecylsulfate, ethanesulfonate, formate, fumarate, glucoheptanoate, glycerophosphate, glycollate, hemisulfate, heptanoate, hexanoate, hydrochloride, hydrobromide, hydroiodide, 2-hydroxyethanesulfonate, lactate, maleate, malonate, methanesulfonate, 2-naphthylsulfonate, nicotinate, nitrate, oxalate, pamoate, pectinate, perchlorate, persulfate, 3-phenylpropionate, phosphate, picrate, pivalate, propionate, salicylate, succinate, sulfate, tartrate, thiocyanate, tosylate, and undecanoate.

Compounds which are encompassed by the present invention also envisions the quaternization of any basic nitrogen containing groups of the compounds disclosed herein. The basic nitrogen may be quaternized with any agents known to those of ordinary skill in the art including, for example, lower alkyl halides, such as methyl, ethyl, propyl and butyl chlorides, bromides and iodides; dialkyl sulfates including dimethyl, diethyl, dibutyl and diamyl sulfates; long chain halides such as decyl, lauryl, myristyl and stearyl chlorides, bromides and iodides, and aralkyl halides including benzyl and phenethyl bromides. Water or oil-soluble or dispersible products may be obtained by such quaternization.

It is to be understood herein, that if a "range" or "group of substances" is mentioned with respect to a particular characteristic (e.g., temperature, concentration, time) of the present invention, the present invention relates to and explicitly incorporates herein each and every specific member and combination of sub-ranges or sub-groups therein whatsoever. Thus, any specified range or group is to be understood as a shorthand way of referring to each and every member of a range or group individually as well as each and every possible sub-ranges or sub-groups encompassed therein; and similarly with respect to any sub-ranges or sub-groups therein. Thus, for example,

- with respect to the number of carbon atoms, the mention of the range of 1 to 6 carbon atoms is to be understood herein as incorporating each and every individual number of carbon atoms as well as sub-ranges such as, for example, 1 carbon atoms, 3 carbon atoms, 4 to 6 carbon atoms, etc.

- with respect to reaction time, a time of 1 minute or more is to be understood as specifically incorporating herein each and every individual time, as well as sub-range, above 1 minute, such as for example 1 minute, 3 to 15 minutes, 1 minute to 20 hours, 1 to 3 hours, 16 hours, 3 hours to 20 hours etc.;

- and similarly with respect to other parameters such as concentrations, elements, etc...

It is in particular to be understood herein that the compound formulae each include each and every individual compound described thereby as well as each and every possible class or sub-group or sub-class of compounds whether such class or sub-class is defined as positively including particular compounds, as excluding particular compounds or a combination thereof; for example an exclusionary definition for the formula (e.g. I) may read as follows: "provided that when one of A and B is -COOH and the other is H, -COOH may not occupy the 4' position".

It is also to be understood herein that "g" or "gm" is a reference to the gram weight unit and "C", or "°C" is a reference to the Celsius temperature unit.

The compounds described herein may easily be prepared using conventional techniques from readily available starting materials. The detailed descriptions of these approaches are presented, for example, in schemes 1 to 5 discussed below.

Scheme 1 illustrates a generic example for the preparation of the phosphate monoester *III* derived from a primary alcohol (see *I*), a compound of HIV protease inhibitors (see example 1 (step G and H) in the experimental portion of this document for a specific example of this synthesis).

Note:

a) $R_2$ and $R_3$ are as defined herein.

The synthesis of phosphate monoester *III* may use a HIV aspartyl protease inhibitor (*I,* see U.S. patent no. 6,632,816) as the starting material. The diethyl phosphotriester *II* was obtained in good yield upon treatment with diethyl chlorophosphate and sodium hydride in a mixture of tetrahydrofuran and triethylphosphate. Then, addition of trimethysilyl bromide in dichloromethane (DCM) gave compound ***III*** in good to excellent yields.

Scheme 1

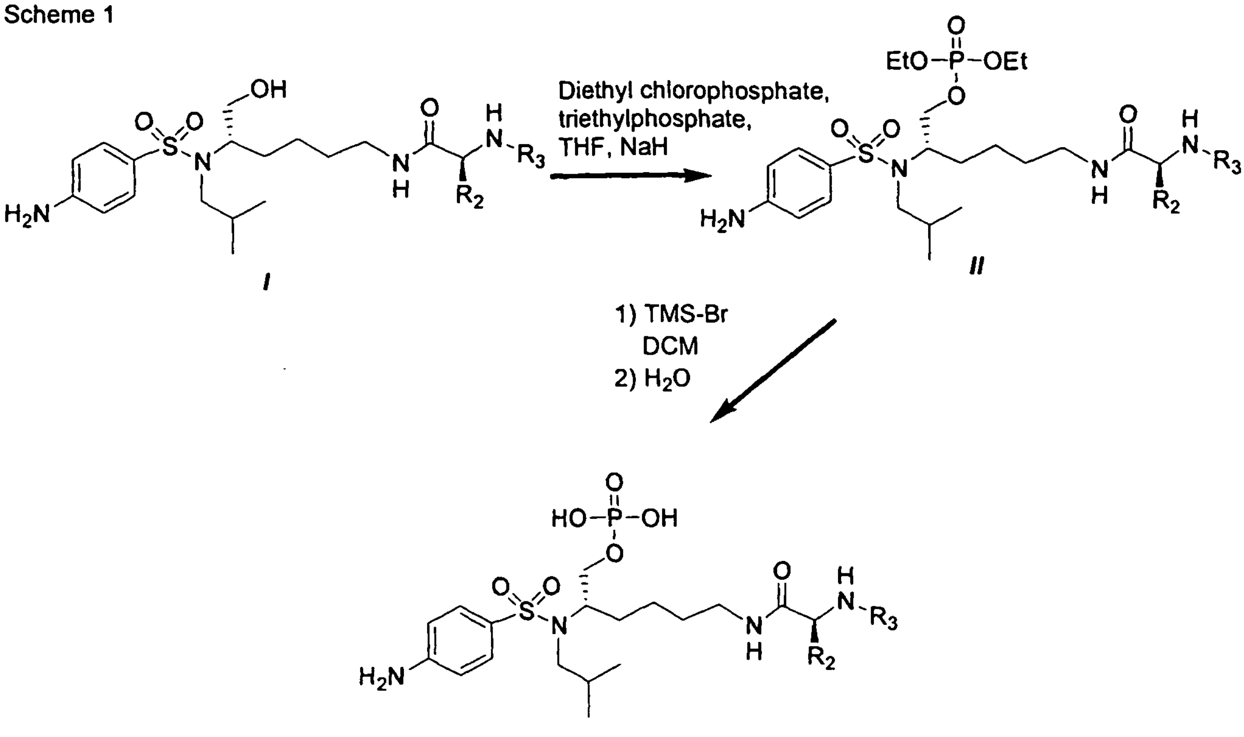


*I*

*II*

*III*

Scheme 1A represents another generic example for the preparation of the phosphate monoester ***IIIA*** derived from a primary alcohol (see ***IA***), a compound of HIV protease inhibitors.

Note:

a) n, X, Y, $R_2$, $R_3$ and $R_6$ are as defined herein.

Scheme 1A

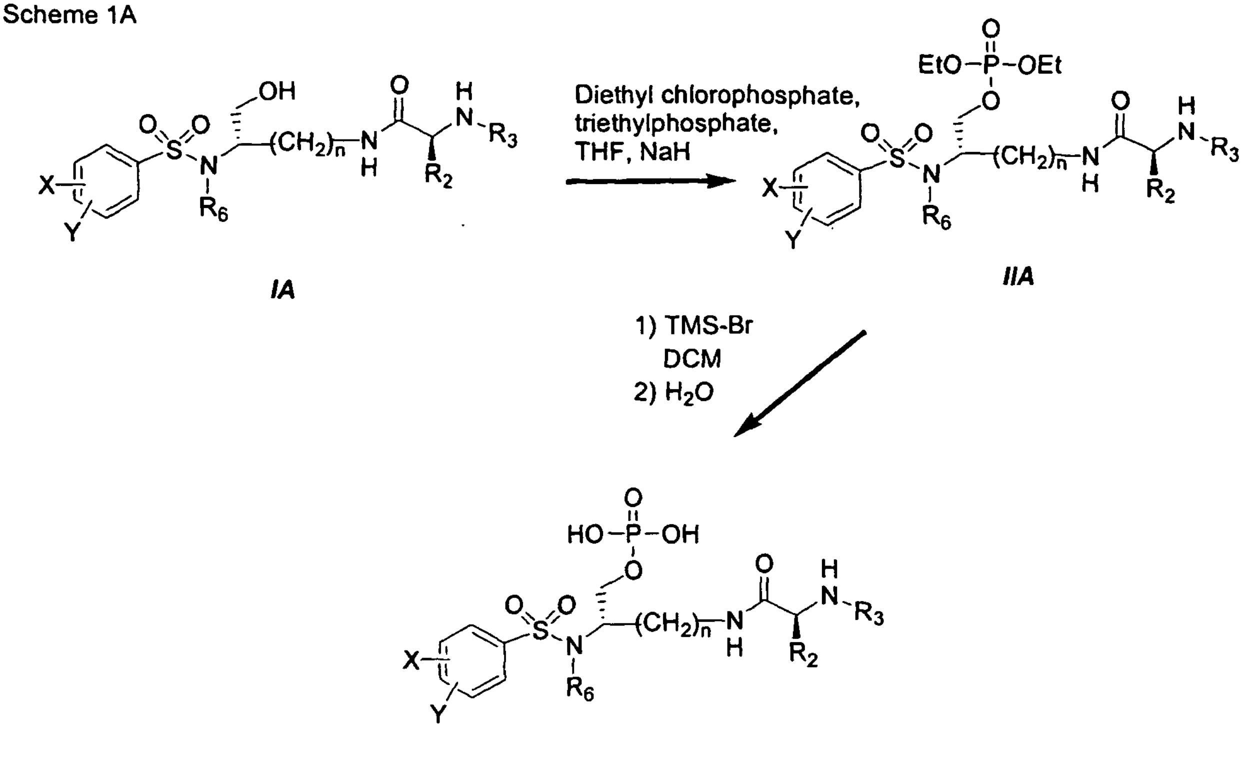


*IA*

*IIA*

*IIIA*

The synthesis of phosphate monoester ***IIIA*** is performed as described for the preparation of ***III*** (scheme 1).

Scheme 2 illustrates a generic example for the preparation of the phosphate monoester ***III,*** a compound of HIV protease inhibitors, with a different approach starting from (3*S*)-3-isobutylamino-azepan-2-one (***IV***).

Note:

a) $R_2$ and $R_3$ are as defined herein.

As shown in scheme 2, the phosphate monoester derivative ***III*** was obtained from (3*S*)-3-isobutylamino-azepan-2-one (***IV***) in a seven-step reaction sequence. Initially, (2*S*)-3-isobutylamino-azepan-2-one (***IV***) was sulfonated with 4-acetamidobenzenesulfonyl chloride in the presence of triethylamine in dichloromethane to give compound ***V*** in excellent yields. The derivative ***VI*** was obtained quantitatively upon treatment of ***V*** with di-*tert*-butyl pyrocarbonate and DMAP in acetonitrile. The reductive ring opening with sodium borohydride in ethanol lead to key intermediates ***VII*** in good yield. The diethyl phosphotriester ***VIII*** was obtained in good yield upon treatment with diethyl chlorophosphate and sodium hydride in a mixture of tetrahydrofuran and triethylphosphate. The Boc protective groups were removed upon treatment with HCl in ethanol to give compound ***IX*** quantitatively (T.W. Greene and P. G. M. Wuts, Protective groups in Organic Synthesis, 3rd Edition, John Wiley & Sons, Inc. 1999). Then, coupling of the free amino group present on intermediate ***IX*** with a variety of synthetic amino acid in the presence of 1-hydroxybenzotriazole (HOBt) and 1-[3-(dimethylamino)propyl]-3-ethylcarbodiimide hydrochloride (EDAC) led to derivative ***II*** in good to excellent yields. Finally, addition of trimethysilyl bromide in dichloromethane (DCM) gave compound ***III*** in good to excellent yields.

Scheme 2

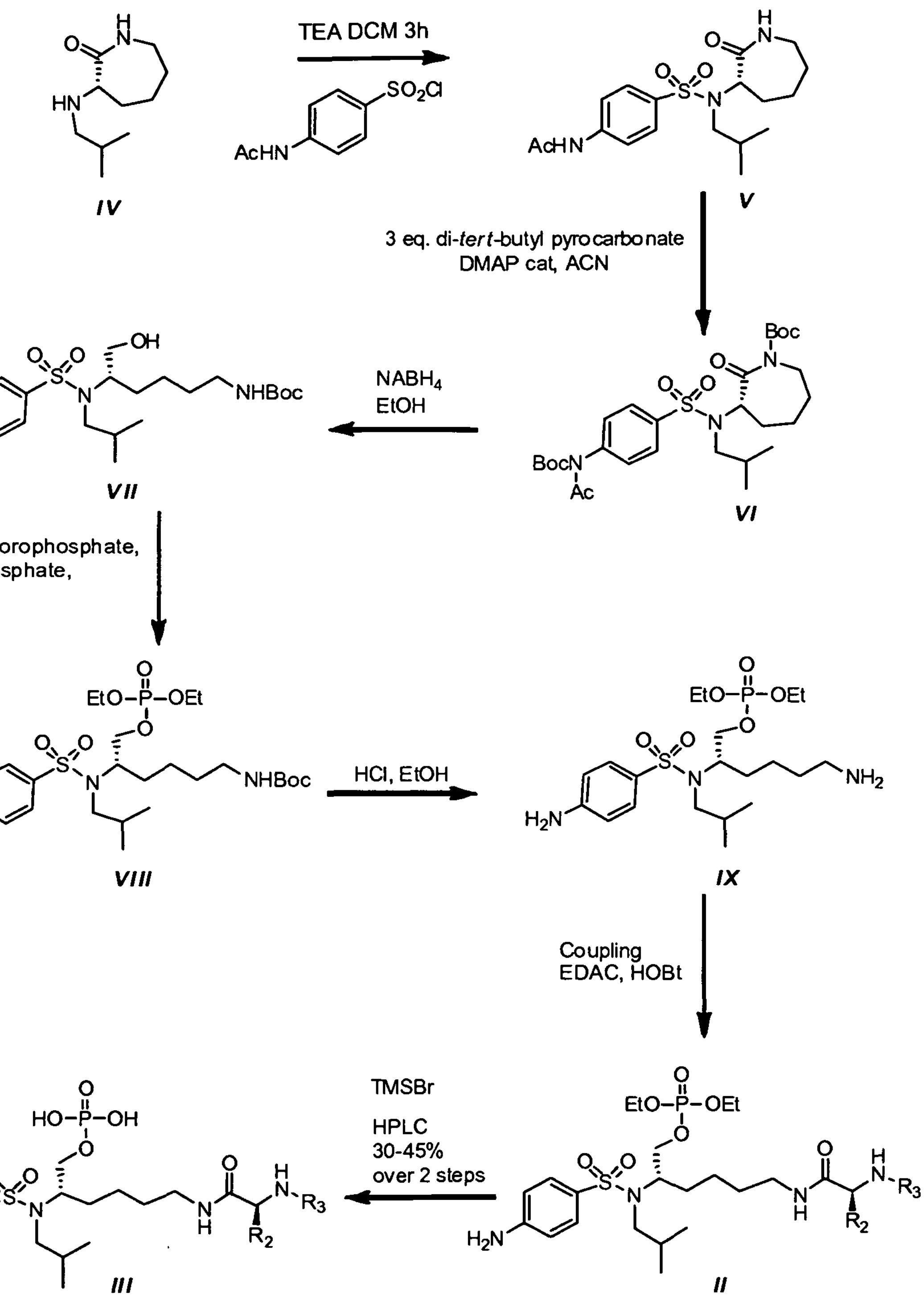


Reference Scheme 3 presents the transformation of a diphenylmethyl derivative; (1S,5S)-(1-{5-[(4-amino-benzenesulfonyl)-isobutyl-amino]-6-hydroxy-hexylcarbamoyl}-2,2-diphenyl-ethyl)-carbamic acid methyl ester **(PL-100)** into its fluorinated phosphate monoester sodium salt analog **XI.** This reaction sequence may be used to produce any other similar compounds (compounds) made of unsubstituted (or substituted) diphenylmethyl, 1-naphthyl, 2-naphthyl, biphenyl and 9-anthryl groups described herein.

Thus, the treatment of **PL-100** with Selectfluor™ in acetonitrile gave derivative **X** in 38% yield. The introduction of the phosphate monoester group was performed as described previously in scheme 1 and 2. First, the diethyl phosphotriester intermediate was obtained in good yield upon treatment with diethyl chlorophosphate and sodium hydride in a mixture of tetrahydrofuran and triethylphosphate. Secondly, addition of trimethysilyl bromide in dichloromethane (DCM) gave the phosphate monoester compound in good to excellent yields. The final product **XI** was easily obtained upon

treatment of the phosphate monoester with a solution of sodium hydroxide with good yields.

Reference Scheme 3

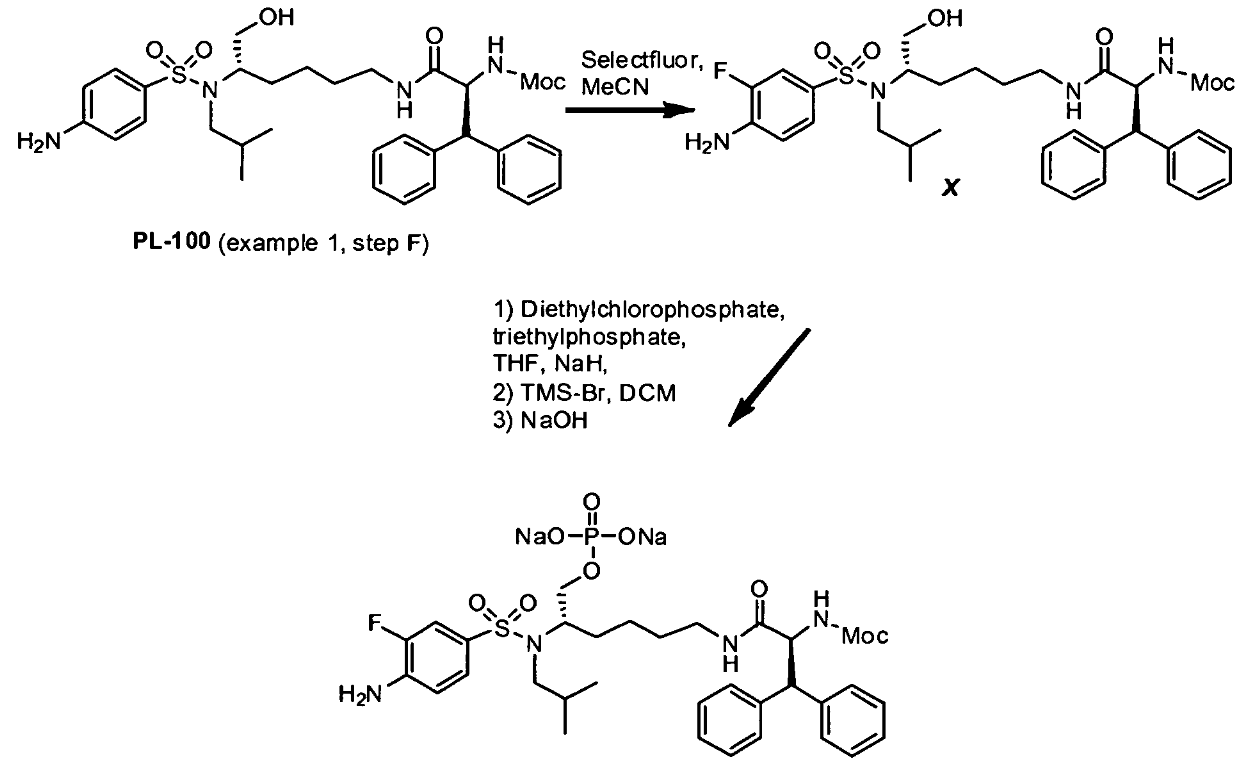


PL-100 (example 1, step F)

X

XI

As it may be appreciated by the person skilled in the art, the above synthetic schemes are not intended to be a comprehensive list of all means by which the compound described in this application may be synthesized but only represent exemplification of synthesis methods among others. Further methods will be evident to those of ordinary skill in the art.

The compounds described herein may be modified by appending appropriate functionalities to enhance selective biological properties. Such modifications may include those which increase biological penetration into a given biological system (e.g., blood, lymphatic system, central nervous system), increase oral availability, increase solubility to allow administration by injection, alter metabolism and alter rate of excretion.

As discussed above, the Lysine-based compounds may release active ingredients which are excellent ligands for aspartyl proteases, for example, HIV-1 protease. Accordingly, these compounds are, by releasing the active ingredient, also capable of targeting and inhibiting late stage events in the replication, i.e. the processing of the viral polyproteins by HIV encoded protease. Compounds described herein advantageously inhibit the ability of the HIV-1 virus to infect immortalized human T cells over a period of days, as determined by an assay measuring the amount of extracellular p24 antigen; a specific marker of viral replication (see, Meek et al., Nature, 343, pp. 90-92 (1990)).

In addition to their use in the prophylaxis or treatment of HIV or HTLV infection, the combination described herein may also be used as inhibitory or interruptive agents for other viruses which use aspartyl proteases, similar to HIV or HTLV aspartyl proteases, in their life cycle. Such compounds inhibit the proteolytic processing of viral polyprotein precursors by inhibiting aspartyl protease. Because aspartyl protease is essential for the production of mature virions, inhibition of that processing effectively blocks the spread of virus by inhibiting the production and reproduction of infectious virions, particularly from acutely and chronically infected cells. The compounds described herein advantageously inhibit aspartyl proteases, thus blocking the ability of aspartyl proteases to catalyze the hydrolysis of peptide bonds.

The combination described herein may be employed in a conventional manner for the treatment or prevention of HIV, HTLV, and other viral infections, which involve aspartyl proteases for their life (replication) cycle. Such methods of treatment, their dosage levels and requirements may be selected by those of ordinary skill in the art from available methods and techniques. For example, combination of a compound as described herein and a CYP450 inhibitor may be associated with a pharmaceutically acceptable adjuvant for administration to a virally infected patient in a pharmaceutically acceptable manner and in an amount effective to lessen the severity of the viral infection.

Alternatively, the combination described herein may be used in vaccines and methods for protecting individuals

against viral infection over an extended period of time. The combination may be employed in such vaccines either alone or together with other compounds of this invention in a manner consistent with the conventional utilization of protease inhibitors or protease inhibitors derivatives in vaccines. For example, combination of a compound as described herein with ritonavir may be combined with pharmaceutically acceptable adjuvants, or delivery systems conventionally employed in vaccines and administered in prophylactically effective amounts to protect individuals over an extended period of time against viral infections, such as HIV infection. As such, the novel compounds of the present invention (upon cleavage of a physiologically cleavable unit) may be administered in combination with ritonavir as therapeutic agents for treating or preventing viral infections, including HIV infection, in a mammal and including AIDS treatment or prevention.

The combination of compounds described herein with ritonavir may be administered to a healthy or HIV-infected patient (before or after the onset of AIDS symptoms) either as a single agent or in combination with other antiviral agents which interfere with the replication cycle of HIV. By administering the compounds of this invention with other antiviral agents which target different events in the viral life cycle, the therapeutic effect of these compounds is potentiated. For instance, the co-administered antiviral agent may be one which targets early events in the viral life cycle, such as attachment to the cell receptor and cell entry, reverse transcription and viral DNA integration into cellular DNA. Antiviral agents targeting such early life cycle events include among others polysulfated polysaccharides, sT4 (soluble CD4) and other compounds which block binding of virus to CD4 receptors on CD4 bearing T-lymphocytes and other CD4(+) cells, or inhibit fusion of the viral envelope with the cytoplasmic membrane, and didanosine (ddl), zalcitabine (ddC), stavudine (d4T), zidovudine (AZT) and lamivudine (3TC) which inhibit reverse transcription. For example another protease inhibitor may be used with compounds of the present invention. Other anti-retroviral and antiviral drugs may also be co-administered with the compounds of this invention to provide therapeutic treatment for substantially reducing or eliminating viral infectivity and the symptoms associated therewith. Examples of other antiviral agents include ganciclovir, dideoxycytidine, trisodium phosphonoformate, eflomithine, ribavirin, acyclovir, alpha interferon and trimenotrexate. Additionally, other types of drugs may be used to potentiate the effect of the compounds of this invention, such as viral uncoating inhibitors, inhibitors of Tat or Rev trans-activating proteins, antisense molecules or inhibitors of the viral integrase. These compounds may also be co-administered with other inhibitors of HIV aspartyl protease. Furthermore, it may be found useful to administer compounds of the present invention with any other drug (other anti-viral compounds, antibiotics, pain killer, etc.).

Combination therapies according to this invention exert a synergistic effect in inhibiting HIV replication because each component agent of the combination acts on a different site of HIV replication. The use of such combinations also advantageously reduces the dosage of a given conventional anti-retroviral agent that would be required for a desired therapeutic or prophylactic effect as compared to when that agent is administered as a monotherapy. These combinations may reduce or eliminate the side effects of conventional single anti- retroviral agent therapies while not interfering with the anti-retroviral activity of those agents. These combinations reduce the potential of resistance to single agent therapies, while minimizing any associated toxicity. These combinations may also increase the efficacy of the conventional agent without increasing the associated toxicity. Combination therapies encompassed by the present invention include, for example, the administration of a compound of this invention with AZT, 3TC, ddl, ddC, d4T or other reverse transcriptase inhibitors.

Alternatively, a combination of compounds described herein with ritonavir may also be co-administered with other HIV protease inhibitors such as Ro 31-8959 (Saquinavir; Roche), L-735,524 (Indinavir; Merck), AG-1343 (Nelfinavir; Agouron), ABT-378/r (Lopinavir; Abbott), and VX-478 (Amprenavir; Glaxo) to increase the effect of therapy or prophylaxis against various viral mutants or members of other HIV quasi species.

Administration of compounds of the present invention may be performed, for example, as single agents or in combination with retroviral reverse transcriptase inhibitors, or other HIV aspartyl protease inhibitors. Co-administration of the compounds of this invention with retroviral reverse transcriptase inhibitors or HIV aspartyl protease inhibitors may exert a substantial synergistic effect, thereby preventing, substantially reducing, or completely eliminating viral infectivity and its associated symptoms.

The compounds of the present invention may be administered in such a manner or form which may allow cleavage of the R1 unit to release a protease inhibitor. The compounds of this invention may also be administered, for example, in combination with immunomodulators (e.g., bropirimine, anti-human alpha interferon antibody, IL-2, GM-CSF, methionine enkephalin, interferon alpha, diethyldithiocarbamate sodium, tumor necrosis factor, naltrexone and rEPO) antibiotics (e.g., pentamidine isethionate) or vaccines to prevent or combat infection and disease associated with HIV infection, such as AIDS and ARC.

When the combination of compounds described herein with ritonavir are administered in combination therapies with other agents, they may be administered sequentially or concurrently to the patient. Alternatively, pharmaceutical or prophylactic compositions according to this invention may be comprised of a combination of one or more compounds of this invention and another therapeutic or prophylactic agent.

Although this invention focuses on the use of the combination disclosed herein for preventing and treating HIV infection, the combination of this invention may also be used as inhibitory agents for other viruses that depend on similar

aspartyl proteases for obligatory events in their life cycle. These viruses include, but are not limited to, retroviruses causing AIDS-like diseases such as simian immunodeficiency viruses, HIV-2, HTLV-I and HTLV-II. In addition, the combination of this invention may also be used to inhibit other aspartyl proteases and, in particular, other human aspartyl proteases including renin and aspartyl proteases that process endothelin precursors.

Pharmaceutical compositions of this invention comprise any of the compounds of the present invention, and pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include, but are not limited to ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethyleneglycol, sodium carboxymethylcellulose, polyacrylates, waxes, polyethylene-polyoxypropylene-block polymers, polyethylene glycol and wool fat.

The pharmaceutical compositions of this invention may be administered orally, parenterally by inhalation spray, topically, rectally, nasally, buccally, vaginally or via an implanted reservoir. It is therefore understood herein that oral administration or administration by injection are encompassed by the present invention. For example, compounds of the present invention, may, for example, be orally administered in an aqueous solution. The pharmaceutical compositions of this invention may contain any conventional non-toxic pharmaceutically acceptable carriers, adjuvants or vehicles. The term "parenteral" as used herein includes subcutaneous, intracutaneous, intravenous, intramuscular, intra-articular, intrasynovial, intrastemal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are amino acid, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv. or a similar alcohol.

The pharmaceutical compositions of this invention may be orally administered in any orally acceptable dosage form including, but not limited to, capsules, tablets, and aqueous suspension and solutions. In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

The pharmaceutical compositions of this invention may also be administered in the form of suppositories for rectal administration. These compositions may be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax, and polyethylene glycols.

Topical administration of the pharmaceutical compositions of this invention is especially useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene or polyoxypropylene compound, emulsifying wax and water. Alternatively, the pharmaceutical compositions may be formulated with a suitable lotion or cream containing the active compound suspended or dissolved in a carrier. Suitable carriers include, but are not limited to, mineral oil, sorbitan monostearate, polysorbate 60, cetyl esters wax cetearyl alcohol, 2-octyldodecanol, benzyl alcohol and water. The pharmaceutical compositions of this invention may also be topically applied to the lower intestinal tract by rectal suppository formulation or in a suitable neat formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline employing benzyl alcohol or other suitable preservatives, absorption promoters to enhance bioavailability, fluorocarbons, and/or other solubilizing or dispersing agents known in the art.

Dosage levels of between about 0.01 and about 150 mg/kg body weight per day of for example, 0.01 and about

50 mg/kg body weight per day or for example from between about 0.5 and about 30 mg/kg body weight per day of the active ingredient compound are useful in the prevention and treatment of viral infection, including HIV infection. Typically, the pharmaceutical compositions of this invention will be administered from about 1 to about 5 times per day or alternatively, as a continuous infusion. Such administration may be used as a chronic or acute therapy. The amount of active ingredient that may be combined with the carrier materials to produce a single dosage form will vary depending upon the patient treated and the particular mode of administration. A typical preparation will contain from about 5% to about 95% of a desired compound (w/w) i.e., active ingredient or a precursor. For example, such preparations may contain from about 20% to about 80% of a desired compound.

Upon improvement of a patient's condition, a maintenance dose of a compound, composition or combination of this invention may be administered if necessary. Subsequently, the dosage or frequency of administration, or both, may be reduced, as a function of the symptoms, to a level at which the improved condition is retained. When the symptoms have been alleviated to the desired level, treatment should cease. Patients may, however, require intermittent treatment on a long-term basis, upon any recurrence of disease symptoms.

As the person skilled in the art will appreciate, lower or higher doses than those recited above may be desired. Specific dosage and treatment regimen for any particular patient may depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician.

In the description herein, the following abbreviations are used:

| Abbreviation | Meaning |
|---|---|
| Ac | Acetyl |
| AcOH | Acetic acid |
| APCI | Atmospheric pressure chemical ionization |
| AIDS | Acquired Immunodeficiency Syndrome |
| APV | Amprenavir |
| ATV | Atazanavir |
| AZT | 3-Azido-3-deoxythymine (Zidovudine) |
| Boc | Benzyloxycarbonyl |
| t-Butyl | *tert*-Butyl |
| CAM | Cerium ammonium molybdate |
| DCM | Dichloromethane |
| DMAP | *N,N*-dimethylaminopyridine |
| DMSO | Dimethylsulfoxide |
| DMF | Dimethylformamide |
| DNA | Deoxyribonucleic acid |
| EDAC | 1-[3-(dimethylamino)propyl]-3-ethylcarbodiimide hydrochloride |
| EtOAc | Ethyl acetate |
| EtOH | Ethyl alcohol |
| g | Gram |
| h | hour |
| HIV-1, -2 | Human immunodeficiency virus type 1, type 2 |
| HOBt | 1-Hydroxybenzotriazole |
| HPLC | High performance liquid chromatography |
| HTLV-I, -II | Human T-cell lymphotropic virus type I, type II |
| IL-2 | Interleukin-2 |
| IDV | Indinavir |
| Kg | Kilogram |
| L | Liter |
| LC-MS | Liquid chromatography-mass spectrometry |
| LPV | Lopinavir |
| M | Molar |
| MeOH | Methyl alcohol |
| mg | Milligram |

(continued)

| Abbreviation | Meaning |
|---|---|
| mp | Melting point |
| min | Minute |
| Moc | Methoxycarbonyl |
| mol | Mole |
| mL | Milliliter |
| mmol | Millimole |
| NFV | Nelfinavir |
| nm | Nanometer |
| nM | Nanomolar |
| po | Orally |
| rEPO | Recombinant erythropoietin |
| RTV | Ritonavir |
| SQV | Saquinavir |
| TLC | Thin layer chromatography |
| 3TC | 2',3'-Dideoxy-3-thiacytidine |
| TFA | Trifluoroacetic acid |
| THF | Tetrahydrofuran |

## BRIEF DESCRIPTION OF THE DRAWINGS

In drawings which illustrates exemplary embodiments of the present invention;

**Fig. 1** is a graph illustrating the fold-change (FC) and median fold-change in $IC_{50}$ vs the reference strain (NL-4.3) observed for various protease inhibitors;

**Fig. 2** is a schematic illustrating the advantages of the Lysine-based compounds disclosed herein;

**Fig. 3** is a schematic illustrating the cleavage of the physiologically cleavable unit of a Lysine-based compound (e.g., PL-461) generating the active ingredient (e.g., PL-100);

**Fig. 4** is a graph illustrating water solubility of PL-461 at various pH;

**Fig. 5** is a graph illustrating the plasma concentration of the drug after administration of either a Lysine-based compound (e.g., PL-461) or an active ingredient (e.g., PL-100) in rats;

**Fig. 6** is a histogram illustrating the effect of CYP450 inhibitors on the metabolism of various HIV-1 protease inhibitors in human liver microsomes;

**Fig. 7** is a histogram illustrating the bioavailability of a Lysine-based compound (e.g., PL-461) or of an active ingredient (e.g., PL-100) in combination with various concentration of a CYP450 inhibitor (e.g., ritonavir) in rats;

**Fig. 8** is a graph illustrating the plasma concentration of the drug after administration of a Lysine-based compound (e.g., PL-461) in combination with a CYP450 inhibitor (e.g., ritonavir) in rats;

**Fig. 9** is a histogram illustrating the bioavailability of the drug following administration of various Lysine-based compound (e.g., PL-461)/CYP450 inhibitor (CYP450) ratios in rats and;

**Fig. 10** is a table illustrating the inhibition of CYP450 sub-families by the active ingredient PL-100.

## EXAMPLES

This section describes the synthesis of lysine based compounds able to release an HIV aspartyl protease inhibitors as described herein. These examples are for the purpose of illustration only and are not to be construed as

limiting the scope of the invention in any way. This section presents the detailed synthesis of compounds no. 1 to 10 of this invention.

Exemplary synthesis schemes of the active ingredients have been disclosed in U.S. patent no. 6,632,816 to Stranix et al,.

**Materials and Methods-preparation of compounds**

Analytical thin layer chromatography (TLC) was carried out with 0.25 mm silica gel E. Merck 60 $F_{254}$ plates and eluted with the indicated solvent systems. Preparative chromatography was performed by flash chromatography, using silica gel 60 (EM Science) with the indicated solvent systems and positive air pressure to allow proper rate of elution. Detection of the compounds was carried out by exposing eluted plates (analytical or preparative) to iodine, UV light and/or treating analytical plates with a 2% solution of *p*-anisaldehyde in ethanol containing 3% sulfuric acid and 1% acetic acid followed by heating. Alternatively, analytical plates may be treated with a 0.3% ninhydrin solution in ethanol containing 3% acetic acid and/or a CAM solution made of 20 g $(NH_4)_6Mo_7O_{24}$ and 8.3 g $Ce(SO_4)_2$ polyhydrate in water (750 mL) containing concentrated sulfuric acid (90 mL).

Preparative HPLC were performed on a Gilson apparatus equipped with a C18 column, a 215 liquid handler module and 25 mL/min capacity head pumps. The HPLC is operated with a Gilson UniPoint System Software.

<u>Semi-preparative HPLC conditions for purification of test compounds:</u>

HPLC system: 2 Gilson #305-25 mL pumps, Gilson #215 liquid handler for injection and collection and a Gilson #155 UV-Vis absorbance detector, all controlled from a Gilson Unipoint V1.91 software
Column: Alltech (#96053) Hyperprep PEP, C-18, 100 Åα, 8 μm, 22 x 250 mm
Flow: 15 mL/min
Solvents: A: $H_2O$; B: $CH_3CN$
Gradient: 25% to 80% of B over 40 min
Detector: absorbance; λ: 210 & 265 nm

The crude material dissolved in acetonitrile to a concentration of around 50 to 80 mg / 2 mL were injected in each run. Fractions were collected in amounts of 9 mL pertaining absorbance was detected at the UV detector.

Unless otherwise indicated, all starting materials were purchased from a commercial source such as Aldrich Co. or Sigma Co.

Melting points (mp) were determined on a Büchi 530 melting point apparatus in capillary tubes and were uncorrected.

Mass spectra were recorded on a Hewlett Packard LC/MSD 1100 system using APCI or electrospray sources either in negative mode or positive mode.

Nuclear magnetic resonance (NMR) spectra were recorded on a Bruker AMX-II-500 equipped with a reversed or QNP probe. Samples were dissolved in deuterochloroform ($CDCl_3$), deuteroacetone (acetone-$d_6$), deuteromethanol ($CD_3OD$) or deuterodimethylsulfoxide (DMSO-$d_6$) for data acquisition using tetramethylsilane as internal standard. Chemical shifts () are expressed in parts per million (ppm), the coupling constants (J) are expressed in hertz (Hz) whereas multiplicities are denoted as s for singlet, d for doublet, 2d for two doublets, dd for doublet of doublets, t for triplet, q for quartet, quint. for quintet, m for multiplet, and br s for broad singlet.

## DETAILED DESCRIPTION OF THE INVENTION

## EXAMPLES:

***<u>Specific examples for the preparation of derivatives of general formula I</u>***

The preparation of PL-100, PL-337 and other compounds of this class is presented in U.S. patent no. 6,632,816 to Stranix et al.

The following compounds were prepared from L-lysine derivatives using the procedures summarized in schemes 1, 1A, 2, 3, 4 and 5 of this invention.

### Example 1. Preparation of (1*S*,5*S*)-(1-{5-[(4-amino-benzenesulfonyl)-isobutyl-amino]-6-phosphonooxy-hexylcarbamoyl}-2,2-diphenyl-ethyl)-carbamic acid methyl ester (PL-461)

The preparation of the title compound is based on schemes 1 and 2 of this invention.

Step A. Preparation of (3*S*)-3-isobutylamino-azepan-2-one (***IV***)

L-$\alpha$-amino-caprolactam (22.0 g) was dissolved in cold dichloroethane (DCM, 200 mL). isobutyraldehyde (12.6 g) was added slowly and stirred until the heat evolved was dissipated (water forms at the surface). The cold solution was added to 46.5 g of powdered $NaBH(OAc)_3$ in DCM (0.5 L). AcOH (70 mL) was added to the solution. The slightly turbid mixture was stirred at 20 °C for 4 h. A 500 mL solution of 2M NaOH was added slowly to the turbid mixture and the pH adjust to 11 using a concentrated NaOH solution, and then the mixture stirred for a further 20 min. After extraction, the DCM layer was dried with $MgSO_4$, filtered and evaporated. The oil thus obtained crystallizes slowly on standing (27.8 g, 85%) and was used without further purification in the next step.

$^1H$ NMR ($CDCl_3$): $\delta$ 0.93 (d, *J* = 6.5, 3H), 0.97 (d, *J* = 6.5, 3H), 1.39 (t, *J* = 9.8, 1 H), 1.47 (m, 1 H), 1.78-1.65 (m, 2H), 2.00-1.93 (m, 2H), 2.32-2.2 (m, 2H), 2.38 (t, *J* = 9.7, 1 H), 3.16 (m, 3H), 6.62 (s, 1 H (NH)). mp 52-54 °C (hexanes).

A small sample was converted to the *S*-methyl benzyl urea by adding the solid to a solution of *S*-methyl benzyl isocyanate in MeCN. NMR gives 98% ee

Step B. Preparation of *N*α-isobutyl-*N*α-(4-acetamidobenzenesulfonyl)-L-$\alpha$-amino-caprolactam (***V***)

*N*α-isobutyl-L-$\alpha$-amino-caprolactam (***IV***) (4.1 g free base) was dissolved in DCM (200 mL) and treated with 4.0 g triethylamine, followed by 4-acetamidobenzenesulfonyl chloride (5.2 g). A 0.1 g portion of dimethylaminopyridine was added and the mixture was stirred 5 h. The resulting thick slurry was poured into 500 mL 0.5 M HCl and shaken vigorously. The solid in the biphasic solution was filtered out and washed with cold acetone to give 7.3 g (87%) of clean product.

$^1H$ NMR (DMSO-$d_6$): 0.93 (d, *J* = 6.0, 3H), 0.96 (d, *J* = 6.0, 3H), 1.39 (t, *J* = 12.0, 1 H), 1.85-1.65 (m, 3H), 2.08-2.18 (m and s, 6H), 2.90-2.97 (m, 1H), 3.00-3.06 (m, 2H), 3.35 (dd, *J* = 14.2, 8.5, 1 H), 4.65 (d, *J* = 8.7, 1H), 6.3 (s, 1 H), 7.42 (d, J = 8.8, 2H), 7.6 (d, *J* = 8.8, 2H). mp 230-233 °C (EtOH).

Step C. Preparation of (3*S*)-3-{[4-(acetyl-tert-butoxycarbonyl-amino)-benzenesulfonyl]-isobutyl-amino}-2-oxo-azepane-1-carboxylic acid tert-butyl ester (Boc activation) (***VI***)

4.2 g of *N*α-isobutyl-*N*α-(4-acetamidobenzenesulfonyl)-L-α-amino-caprolactam (***V***) was suspended in 30 mL MeCN and briefly sonicated to break up any large chunks. To this white suspension was added 6.7 g (3 eq.) of di-*tert*-butyl pyrocarbonate in 10 mL MeCN. The suspension was stirred with a magnetic bar and a 120 mg portion of DMAP was added. The solution becomes a clear light yellow after a few minutes. TLC (EtOAc) reveals 1 product Rf 0.9 (starting material Rf at 0.4). The solution is poured in distilled water 20 mL and extracted with ether, dried with $Na_2SO_4$ and evaporated yielding 6.90 g. A sample was recrystallized from hexanes.

$^1H$ NMR (DMSO-$d_6$): 0.68 (d, *J* = 6.0, 3H), 0.85 (d, *J* = 6.0, 3H), 1.39 (s, 10H), 1.47 (s, 9H), 1.85-1.65 (m, 3H), 2.15 (s, 3H), 2.80 (q, *J* = 4, 1 H), 3.10-3.36 (m, 2H), 4.01 (d, *J* = 8.0, 1 H), 4.85 (d, *J* = 8.7, 1 H), 7.32 (d, *J* = 8.8, 2H), 7.87 (d, *J* = 8.8, 2H). mp 123-124 °C

Step D. Preparation of (1*S*)-4-amino-*N*-(5-amino-1-hydroxymethyl-pentyl)-*N*-isobutyl-benzenesulfonamide **(*VII*-deprotected)** (reductive ring opening and deprotection)

A 3.0 g portion of (3*S*)-3-{[4-(acetyl-tert-butoxycarbonyl-amino)-benzenesulfonyl]-isobutyl-amino}-2-oxo-azepane-1-carboxylic acid tert-butyl ester **(*VI,*** step C) is dissolved in 40 mL EtOH followed by 750 mg $NaBH_4$. Brief heating with a heat gun gives a clear solution. TLC reveals one streaky spot after 20 min (EtOAc). The solution is concentrated to a paste, poured in 40 mL 1 N NaOH and extracted with ethyl acetate, the organic phase dried with $NaSO_4$ and evaporated to give 2.8 g of product intermediate (***VII***); (1*S*)-{4-[(5-tert-butoxycarbonylamino-1-hydroxymethyl-pentyl)-isobutyl-sulfamoyl]-phenyl}-carbamic acid tert-butyl ester (***VII***).

The above product intermediate is dissolved in 5 mL EtOH and 5 mL 12 N HCl is added. Vigorous gas evolution is observed for a few minutes. After 2 h the solution is evaporated and rendered basic with concentrated KOH and extracted with EtOAc yielding 1.75 g of a white powder.

$^1H$ NMR (DMSO-$d_6$): 0.82 (m, 6H), 0.97-1.12 (m, 2H), 1.15-1.30 (m, 3H), 1.57 (m, 1H), 1.84 (m, 1 H), 2.40 (t, *J* = 7.8, 2H), 2.75 (m, 1 H), 2.85 (m, 1 H), 3.21 (m, 1 H), 3.44 (d, *J* = 6.4, 2H), 5.92 (br s, 2H), 6.59 (d, *J* = 8.0, 2H), 7.39 (d, *J* = 8.0, 2H).

Step E. Preparation (2*S*)-2-methoxycarbonylamino-3,3-diphenyl-propionic acid

To a solution of L-diphenylalanine (241 mg, 1.0 mmol) (Peptech Corp.) in 5 mL 1N NaOH and 0.5 mL saturated $Na_2CO_3$ (resulting solution at pH 10) was added methoxycarbonyloxysuccinimide (carbonic acid 2,5-dioxo-pyrrolidin-1-yl ester methyl ester) (180 mg, 1.1 mmol) dissolved in 5 mL. Afterwards, the reaction mixture was stirred at room

temperature for 2 h. The alkaline solution was extracted once with ether (10 mL) and the aqueous phase was acidified with 1 N HCl. This was extracted twice with 20 mL EtOAc, and the combined organic phases were washed with 50 mL 1 N HCl. The organic phase was dried over $Na_2SO_4$. filtered and evaporated to an oil, which solidifies to yields 250 mg (83%) of the desired material. This derivative was used as such in the next step.

Step F. Preparation of (1*S*,5*S*)-(1-{5-[(4-amino-benzenesulfonyl)-isobutyl-amino]-6-hydroxy-hexylcarbamoyl}-2,2-diphenyl-ethyl)-carbamic acid methyl ester (PL-100)

The title compound was prepared from (1*S*)-4-amino-*N*-(5-amino-1-hydroxymethyl-pentyl)-*N*-isobutyl-benzenesulfonamide **(*VII*-deprotected)** (step D) and (2*S*)-2-methoxycarbonylamino-3,3-diphenyl-propionic acid (step E) using the coupling procedure with HOBt and EDAC described in example 3 (step D). The final product was obtained in 67% yield (121 mg).

LC-MS : 625.3 $(M+H)^+$, 95% pure

$^1H$ NMR ($CD_3OD$): $\delta$ 0.71-0.85 (m, 2H), 0.88 (d, *J* = 6.3, 5H), 0.91-0.96 (m, 2H), 1.29-1.34 (m, 1H), 1.41-1.52 (m, 1H) 1.82-1.92 (m, 1H), 2.61-2.68 (m, 1H), 2.81-2.85 (m, 2H), 2.94-3.05 (m, 2H), 3.38-3.40 (t, *J* = 5.0, 1 H), 3.50-3.51 (m, 1H), 3.52 (s, 3H), 4.28 (d, *J* = 11.0 1 H), 4.87 (d, *J* = 11.0, 1 H), 6.69 (d, *J* = 8.0, 2H), 7.15-718 (m, 2H), 7.20-7.31 (m, 6H), 7.33 (d, *J* = 7.9, 2H), 7.47 (d, *J* = 7.5, 1 H).

$^{13}C$ NMR ($CD_3OD$): $\delta$ 20.0, 20.1, 23.3, 25.4, 28.1, 28.5, 28.9, 38.1, 40.0, 51.2, 51.6, 53.1, 57.2, 57.4, 59.5, 61.9, 62.4, 112.6, 125.7, 126.2, 126.3, 127.9, 128.1,128.15, 128.2, 128.4, 128.7, 141.3, 141.9, 152.4, 155.9, 169.9, 172.5.

Step G. Preparation of (1*S*,5*S*)-{1-[5-[(4-amino-benzenesulfonyl)-isobutyl-amino]-6-(diethoxy-phosphoryloxy)-hexylcarbamoyl]-2,2-diphenyl-ethyl}-carbamic acid methyl ester

The PL-100 compound (product of step F, 203 mg, 0.325 mmol) was dissolved in dry tetrahydrofuran (3 mL) and 0.2 mL triethylphosphate under $N_2$ atmosphere. The mixture was stirred at this temperature for 15 min, followed by the addition of diethyl chlorophosphate (0.061 mL, 0.423 mmol). Sodium hydride (60% in mineral oil) (17 mg, 0.423 mmol) was added at 0 °C. The solution was stirred for 1 h at 0 °C and 12 h at room temperature. 20 mL of Amberlite XAD-2 was added to the solution and the beads were thoroughly mixed with the solvent. To the mixture was added ice water 2 mL, and the THF evaporated off. The beads were then washed with distilled water 6 times 100 mL then extracted three times with ethyl acetate (30 mL). The combined phase was evaporated and the residue was dried under high vacuum. The crude product was purified by flash chromatography using ethyl acetate/hexane (8/2), then EtOAc 100% as eluent. The yield of this reaction is 152 mg 61%.

LC-MS: 761.2 $(M+H)^+$, 90% pure

$^1H$ NMR ($CD_3OD$): $\delta$ 0.68-0.75 (m, 1H), 0.75-0.84 (m, 1H), 0.84-1.10 (m, 9H), 1.21-1.50 (m, 8H), 1.88 (m, 1 H), 2.58-2.71 (m, 1H), 2.80-2.89 (m, 1 H), 2.89-3.08 (m, 2H), 3.49-3.60 (s, 3H), 3.65-3.74 (m, 1H), 3.85-3.95 (m, 1H), 3.97-4.02 (m, 1H), 4.07-4.21 (m, 4H), 4.29 (d, J = 10.8, 1H), 6.71 (d, J = 8.0, 2H), 7.10-7.20 (m, 2H), 7.20-7.32 (m, 5H), 7.32-7.45 (m, 3H), 7.50 (d, J = 7.5, 2H), 7.86 (br s, 1H).

$^{31}P$ NMR ($CD_3OD$): $\delta$ 1.62

Step H. Preparation of (1*S*,5*S*)-(1-{5-[(4-amino-benzenesulfonyl)-isobutyl-amino]-6-phosphonooxy-hexylcarbamoyl}-2,2-diphenyl-ethyl)-carbamic acid methyl ester (PL-461)

The product of step G prepared above (152 mg) was dissolved in anhydrous dichloromethane (3.0 mL). Trimethylsilyl bromide (0.5 mL) was added at 0 °C. The mixture was stirred during 1 h at this temperature and overnight at room temperature. The solvent was evaporated and 0.2 mL water was added to the residue. 3 mL EtOH was added mixed and evaporated. This step was repeated three times and the residue dried in vacuo. Yields 98 mg 70% of the title derivatives of this first example.

LC-MS: 705.2 $(M+H)^+$, 95% pure

$^1H$ NMR ($CD_3OD$): $\delta$ 0.65-0.73 (m, 1H), 0.75-0.83 (m, 1H), 0.89 (d, J = 5.6, 8H), 1.27-1.38, (m, 1H), 1.42-4.55 (m, 1H), 1.82-1.94 (m, 1H), 2.57-2.68 (m, 1H), 2.78-2.90 (m, 1H), 2.91-3.09 (m, 2H), 3.54 (s, 3H), 3.60-3.72 (m, 1H), 3.87-4.05 (m, 1H), 4.00 (m, 1H), 4.29 (d, J = 11.3, 1H), 4.90 (d, J = 11.4, 1 H), 6.73 (d, J = 8.0, 2H), 7.13-7.22 (m, 2H), 7.22-7.33 (m, 6H), 7.33-7.45 (m, 2H), 7.51 (d, J = 7.5, 2H).

$^{31}P$ NMR ($CD_3OD$): $\delta$ 2.80

**Example 2. Preparation of (1*S*,5*S*)-(1-{5-[(4-amino-benzenesulfonyl)-isobutyl-amino]-6-phosphonooxy-hexylcarbamoyl}-2,2-diphenyl-ethyl)-carbamic acid methyl ester sodium salt (PL-462)**

70.7 mg of the final product of example 1 is added to 1 mL 0.1 N NaOH and diluted with 1 mL of distilled water.

The Solution is then frozen and lyophilized. Yields 67.2 mg (92%) of the desired material with 95% purity.

$^1H$ NMR ($CD_3OD$): $\delta$ 0.72-0.83 (m, 1H), 0.90 (d, J = 5.8, 9H), 1.26-1.38 (m, 1H), 1.53-1.65 (m, 1H), 1.88-2.00 (m, 1H), 2.60-2.70 (m, 1H), 2.79-2.89 (m, 1H), 2.98-3.00 (m, 1H), 3.00-3.08 (m, 1 H), 3.54 (s, 3H), 3.58-3.71 (m, 1 H), 3.72-3.83 (m, 1 H), 3.84-3.95 (m, 1 H), 4.28 (d, J = 11.1, 1H), 4.91 (d, J = 11.0, 1H), 6.70 (d, J = 7.6, 2H), 7.12-7.22 (m, 2H), 7.22-7.32 (m, 6H), 7.33-7.40 (m, 2H), 7.50 (d, J = 7.7, 2H).

$^{31}P$ NMR ($CD_3OD$): $\delta$ 3.13

**Reference Example 3. Preparation of (1*S*,5*S*)-(1-{5-[(4-amino-benzenesulfonyl)-isobutyl-amino]-6-phosphonooxy-hexylcarbamoyl}-2-naphthalen-2-yl-ethyl)-carbamic acid methyl ester (PL-507)**

The preparation of the title compound is based on scheme 2 of this invention.

Step A. Preparation of (1*S*)-(4-{[5-tert-butoxycarbonylamino-1-(diethoxyphosphoryloxymethyl)-pentyl]-isobutyl-sulfamoyl}-phenyl)-carbamic acid tert-butyl ester (***VIII***)

2.00 g (3.7 mmol) (1*S*)-{4-[(5-tert-butoxycarbonylamino-1-hydroxymethyl-pentyl)-isobutyl-sulfamoyl]-phenyl}-carbamic acid tert-butyl ester (***VII***) (example 1, step D) is dissolved in 0.63 mL triethylphosphate and 10 mL THF at 0 °C under inert argon atmosphere. 0.63 mL (4.44 mmol) diethylchlorophosphate is added and then 0.25 g (6.2 mmol), NaH 60% in oil is added in portionwise. The mixture is allowed to warm to room temperature and left to stir for 2 h (LC-MS showed completion after 1 h). To the solution is added 20 mL of Amberlite XAD-2 resin and the slurry thoroughly mixed and added to 200 mL ice water. After stirring for 15 min. the resin suspension is filtered and the resin washed several times with distilled water (500 mL). The desired product is desorbed from the resin with acetone (5 X 50 mL), EtOAc (5 X 50 mL), the organic phase is then dried over $Na_2SO_4$. After evaporation of the solvent 2.66 g (89%) of clear oil is obtained. The crude product contains a fraction with two diethyl phosphates and is used as is in the next step.

$^1H$ NMR ($CD_3OD$): $\delta$ 0.91 (d, J = 6.3, 6H), 1.11-1.21 (m, 2H), 1.33 (t, J = 6.9, 10H), 1.43 (s, 9H), 1.53 (s, 10H), 1.90-1.97 (m, 1H), 2.88-2.96 (m, 3H), 2.96-3.04 (m, 1H), 3.81-3.90 (m, 1 H), 3.91-3.99 (m, 1 H), 4.01-4.14 (m, 4H), 7.61 (d, J = 8.3, 2H), 7.72 (d, J = 8.4, 2H).

$^{31}P$ NMR ($CD_3OD$): $\delta$ 1.59

Step B. Preparation of (2*S*)-phosphoric acid 6-amino-2-[(4-amino-benzenesulfonyl)-isobutyl-amino]-hexyl ester diethyl ester (***IX***)

The crude product obtained in the previous step (***VIII***, 2.66 g) is dissolved in 12 mL EtOH. 4 mL of $HCl_{conc.}$ is added and heated briefly to 70 °C then left at room temperature for 3h. The solvent is evacuated and the residue triturated with 50 mL ether. The thick residue is then dissolved in 3 mL ice water and the pH adjusted to 12 with 50% NaOH. The thick slurry obtained is extracted with EtOAc (3 X 50 mL) and the organic phase dried over $Na_2SO_4$. After filtration of the drying agent the organic phase is evacuated to yield 1.84 g (98%) of the desired product (***IX***).

LC-MS: 480.2 $(M+H)^+$, 95% pure.

$^1H$ NMR ($CD_3OD$): $\delta$ 0.91 (s, 6H), 1.11-1.26 (m, 3H), 1.28-1.43 (m, 8H), 1.45-1.51 (m, 1H), 1.52-1.61 (m, 1H), 1.89-1.96 (m, 1H), 2.56 (t, J = 6.7, 2H), 2.85-2.91 (m, 1H), 2.98-3.11 (m, 1H), 3.79-3.99 (m, 1 H), 3.94 (d, J = 5.3, 1 H), 4.09-4.11 (m, 4H), 6.69 (d, J = 7.9, 2H), 7.50 (d, J = 7.9, 2H).

$^{31}P$ NMR ($CD_3OD$): $\delta$ 1.61

Step C. Preparation of (2*S*)-2-methoxycarbonylamino-3-naphthalen-2-yl-propionic acid (or L-Moc-2-naphthylalanine)

To a solution of L-2-naphthylalanine (215 mg, 1 mmol) (Peptech Corp.) in 5 mL 1N NaOH and 0.5 mL saturated $Na_2CO_3$ (resulting solution at pH 10) was added methoxycarbonyloxysuccinimide (187 mg, 1.1 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1 N HCl. The organic phase was dried over $Na_2SO_4$, filtered and evaporated to an oil, which solidifies to yields 200 mg (73%) of the desired material. This intermediate (referred as the *N*-substituted amino acid) was used without further purification in the next step.

Step D. Preparation of (1*S*,5*S*)-(1-{5-[(4-amino-benzenesulfonyl)-isobutyl-amino]-6-phosphonooxy-hexylcarbamoyl}-2-naphthalen-2-yl-ethyl)-carbamic acid methyl ester (PL-507)

100 mg L-Moc-2-naphthylalanine (step C) was activated with 100 mg EDAC and 57 mg HOBt in 1.5 mL DMF for 30 minutes. Then, 100 mg of phosphoric acid 6-amino-2-[(4-amino-benzenesulfonyl)-isobutyl-amino]-hexyl ester

diethyl ester (step B) was added and left to stir at room temperature for 1 h. 40 mL of 1M $K_2CO_3$ was added to the DMF solution and left for 10 min. 50 mL of EtOAc was then added and the mixture was then agitated vigorously. Separation of the EtOAc phase was effected, followed by extraction with 5% citric acid (50 mL) once, then water (50 mL) 3 times and finally brine. The organic phase was the separated and evaporated. The residue was taken up in 50 mL DCM and re-evaporated. The residue was again taken up in 50 mL DCM and 0.5 mL of TMSBr was added. The solution was left overnight (16 h). The DCM was evacuated and a solution of ice cold MeOH: Water 1:1 was added, stirred briefly and evacuated. The residue was triturated with ether then dissolved in 1N NaOH. The clear solution was extracted with ether and the aqueous phase acidified with 6N HCl. The white precipitated was then collected by filtration and dried in vacuo overnight. Yields 88 mg of the title compound.

LC-MS: 679.8 $(M+H)^+$, 95% pure.

$^1H$ NMR ($CD_3OD$): $\delta$ 0.89-0.98 (m, 8H), 1.15 (m, 2H), 1.35 (m, 1H), 1.45 (m, 1H), 1.88 (m, 1H), 2.84 (m, 2H), 2.98 (m, 1H), 3.01 (m, 2H), 3.24 (m, 1H), 3.56 (s, 3H), 3.60 (m, 1H), 3.81 (m, 1H), 3.99 (m, 1 H), 4.39 (t, J = 6.8, 1 H), 6.91 (d, J = 8.0, 2H), 7.34 (d, J= 8.0, 1 H), 7.45 (m, 2H), 7.58 (d, J = 8.0, 2H), 7.66 (s, 1 H), 7.70-7.82 (m, 3H).

$^{31}P$ NMR ($CD_3OD$): $\delta$ 2.56

**Reference Example 4. Preparation of (2*S*,2*S*) phosphoric acid mono-(2-[(4-aminobenzenesulfonyl)-isobutyl-amino]-6-{2-[(morpholine-4-carbonyl)-amino]-3-naphthalen-1-yl-propionylamino}-hexyl) ester (PL-498)**

Step A. Preparation of (2*S*)-2-[(morpholine-4-carbonyl)-amino]-3-naphthalen-1-yl-propionic acid

To a solution of L-1-naphthylalanine (215 mg, 1 mmol) (Peptech Corp.) in 5 mL 1N NaOH and 0.5 mL saturated $Na_2CO_3$ (resulting solution at pH 10) was added morpholine-4-carbonyl chloride (150 mg, 1.0 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1 N HCl. The organic phase was dried over $Na_2SO_4$, filtered and evaporated to an oil, which solidifies to yields 125 mg (38%) of the desired material. This compound was used as such in the next step.

Step B. Preparation of (2*S*,2*S*) Phosphoric acid mono-(2-[(4-amino-benzenesulfonyl)-isobutyl-amino]-6-{2-[(morpholine-4-carbonyl)-amino]-3-naphthalen-1-yl-propionylamino}-hexyl) ester (PL-498)

This compound was made as for the preparation of the product of example 3 (step D) with 100 mg of (2*S*)-2-[(morpholine-4-carbonyl)-amino]-3-naphthalen-1-yl-propionic acid (step A of this example). The resulting precipitated residue was further purified by reverse phase preparative HPLC. Yields 41 mg of the final compound.

LC-MS: 734.8 $(M+H)^+$, 95% pure.

$^1H$ NMR ($CD_3OD$): $\delta$ 0.83-0.98 (m, 8H), 1.00-1.25 (m, 4H), 1.45-1.52 (m, 1H), 1.52-1.66 (m, 1 H), 1.88-1.99 (m, 1H), 2.77-2.92 (m, 2H), 2.98-3.16 (m, 3H), 3.40-3.49 (m, 1H), 3.50-3.56 (m, 6H), 3.67-3.69 (m, 1H), 3.81-3.89 (m, 1H), 3.99-4.05 (m, 1H), 4.59 (t, J = 6.0, 1H), 6.75 (d, J = 8.0, 2H), 7.30-7.60 (m, 7H), 7.75 (d, J = 8.0, 1H), 7.90 (d, J= 7.8, 1H), 8.23 (d, J=7.8 2H).

$^{31}P$ NMR ($CD_3OD$): $\delta$ 2.71

**Example 5. Preparation of (2*S*,2*S*)-phosphoric acid mono-{6-(2-acetylamino-3,3-diphenyl-propionylamino)-2-[(4-amino-benzenesulfonyl)-isobutyl-amino]-hexyl} ester (PL-504)**

Step A. Preparation (2S)-2-acetylamino-3,3-diphenyl-propionic acid

To a solution of L-diphenylalanine (100 mg, 0.4 mmol) (Peptech Corp.) in 5 mL 1N NaOH and 0.5 mL saturated $Na_2CO_3$ (resulting solution at pH 10) was added acetyl chloride (0.5 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1N HCl. The organic phase was dried over $Na_2SO_4$, filtered and evaporated to an oil, which solidifies to yields 70 mg (60%) of the desired material. This crude intermediate was used as such in the next step.

Step B. Preparation of (2S,2S)-phosphoric acid mono-{6-(2-acetylamino-3,3-diphenyl-propionylamino)-2-[(4-amino-benzenesulfonyl)-isobutyl-amino]-hexyl} ester (PL-504)

This compound was made as for the preparation of the product of example 3 (step D) with 100 mg of (2*S*)-2-acetylamino-3,3-diphenyl-propionic acid (this example step A). The final product was obtained in 30% yield (30 mg).

LC-MS: 689.3 $(M+H)^+$, 95% pure.

$^1H$ NMR ($CD_3OD$): $\delta$ 0.77-1.04 (m, 9H), 1.10-1.17 (m, 1 H), 1.23-1.49 (m, 1 H), 1.46-1.57 (m, 1H), 1.78 (s, 3H), 1.88-1.99 (m, 1H), 2.80-2.92 (m, 2H), 2.92-3.08 (m, 2H), 3.63-3.75 (m, 1H), 3.79-3.95 (m, 1H), 4.00 (m, 1H), 4.34 (d, J = 11.3, 1H), 5.19-5.28 (m, 1H), 6.77-6.85 (m, 2H), 7.10-7.20 (m, 2H), 7.27-7.33 (m, 6H), 7.32-7.41 (m, 2H), 7.49-7.62 (m, 2H).

$^{31}P$ NMR ($CD_3OD$): $\delta$ 2.70

**Reference Example 6. Preparation of (1*S*,5*S*)-(1-{5-[(4-amino-3-fluoro-benzenesulfonyl)-isobutyl-amino]-6-phosphonooxy-hexylcarbamoyl}-2,2-diphenyl-ethyl)-carbamic acid methyl ester (PL-515)**

__**First methodology**__: The preparation of the title compound is based on scheme 3 of this invention.

Step A. Preparation of (1-{5-[(4-amino-3-fluoro-benzenesulfonyl)-isobutyl-amino]-6-hydroxy-hexylcarbamoyl}-2,2-diphenyl-ethyl)-carbamic acid methyl ester (X) (PL-337)

The product of example 1, step F (0.624 g, 1 mmol) is dissolved in 5 mL MeCN at 24 °C. SelectFluor 0.35 g (1 mmol) is added in one portion and stirred for 1 h. 1 mL of water is added and the solution was injected directly into a preparative reverse-phase HPLC. The product was collected and lyophilized to give 250 mg (38%) yield of a white solid.

LC-MS: 643.3 $(M+H)^+$, 99% pure.

$^1H$ NMR (MeOD): $\delta$ 0.71-0.85 (m 2H), 0.88 (d, J = 6.3, 6H), 0.91-0.96 (m, 2H), 1.21-1.29 (m, 1 H), 1.41-1.52 (m, 1H) 1.82-1.92 (m, 1 H), 2.61-2.68 (m, 1 H), 2.81-2.85 (m, 2H), 2.94-3.05 (m, 2H), 3.38-3.40 (t, J = 5, 1 H), 3.49-3.52 (m, 5H), 4.28 (d, J = 10, 1 H), 4.87 (d, J = 10, 1 H) 6.90 (t, J = 8.3, 1H), 7.20 (m, 2H), 7.28 (m, 3H), 7.33 (m, 3H), 7.39 (m, 4H).

Step B. Preparation of (1*S*,5*S*)-{1-[5-[(4-amino-3-fluoro-benzenesulfonyl)-isobutyl-amino]-6-(diethoxy-phosphoryloxy)-hexylcarbamoyl]-2,2-diphenyl-ethyl}-carbamic acid methyl ester

The product of step A was phosphorylated with chlorodiethylphosphate following the procedure described in example 1, step G. Yields 157 mg, 68%.

LC-MS: 779.3 $(M+H)^+$, 95% pure.

$^1H$ NMR ($CD_3OD$): $\delta$ 0.82 (m, 1H), 0.92 (d, J = 6.2, 8H), 0.96 (m, 3H), 1.36 (d, J = 3.7, 6H), 1.90 (m, 1H), 2.69 (m, 1 H), 2.89 (m, 1H), 2.98 (m, 2H), 3.56 (s, 3H), 3.74 (m, 1H), 3.93 (m, 1 H), 4.03 (m, 1 H), 4.12 (q, J = 7.5 and 14.8, 4H), 4.32 (d, J = 11.4, 1 H), 4.92 (d, J = 11.4, 1 H), 6.90 (t, J = 8.3, 1H), 7.20 (m, 2H), 7.28 (m, 3H), 7.33 (m, 3H), 7.39 (m, 4H).

$^{31}P$ NMR ($CD_3OD$): $\delta$ 1.65

Step C. Preparation of (1*S*,5*S*)-(1-{5-[(4-amino-3-fluoro-benzenesulfonyl)-isobutyl-amino]-6-phosphonooxy-hexylcarbamoyl}-2,2-diphenyl-ethyl)-carbamic acid methyl ester (***XI***) (PL-515)

Deprotection was effected using the procedure described in example 1, step G. Yields 101 mg.

LC-MS: 723.2 $(M+H)^+$, 95% pure.

$^1H$ NMR ($CD_3OD$): $\delta$ 0.65-0.77 (m, 1 H), 0.77-0.85 (m, 1H), 0.85-1.05 (m, 9H), 1.25-1.39 (m, 1 H), 1.40-1.52 (m, 1H), 1.82-1.98 (m, 1 H), 2.58-2.72 (m, 1H), 2.82-2.92 (m, 1H), 2.92-3.05 (m, 2H), 3.54 (s, 3H), 3.64-3.75 (m, 1 H), 3.80-3.92 (m, 1H), 3.91-4.04 (m, 1 H), 4.29 (d, J = 11.4, 1H), 7.19 (t, J = 6.6, 1H), 7.13-7.21 (m, 2H), 7.22-7.33 (m, 6H), 7.34-7.38 (m, 2H), 7.39-7.48 (m, 2H).

$^{31}P$ NMR ($CD_3OD$): $\delta$ 2.74

__**Second methodology**__: The preparation of the title compound is based on scheme 4 of this invention.

Step A. Preparation (1*S*,5*S*)-(1-{5-[(4-amino-benzenesulfonyl)-isobutyl-amino]-6-phosphonooxy-hexylcarbamoyl}-2,2-diphenyl-ethyl)-carbamic acid methyl ester (PL-461)

(2*S*)-2-methoxycarbonylamino-3,3-diphenyl-propionic acid ((example 1, step E) 0.9 g, 3 mmol) was activated in DMF (5 mL) with EDAC (1.7 g, 9 mmol) and HOBt (1.2 g, 9 mmol). To the solution was added 1.17 g of (2*S*)-phosphoric acid 6-amino-2-[(4-amino-benzenesulfonyl)-isobutyl-amino]-hexyl ester diethyl ester (***IX***) (example 3, step B) and the mixture stirred for 3 h. 20 g of Amberlite XAD-2 resin was then added and the beads were left to soak for 10 min. The resin was transferred into a glass filter and washed thoroughly with distilled water (400 mL) and 200 mL of 1 M $NaHCO_3$. The beads were then washed with 4 X 50 ml portions of MeOH then EtOAc 200 mL. The organic phase was evaporated. The residue was adsorbed onto silica gel and passed through a short silica gel column (EtOAc) to yield 2.4 g (83%) of white solid after evaporation.

NMR identical as in example 1, step H.

Step B. Preparation (1*S*,5*S*)-{1-[5-[(4-amino-3-fluoro-benzenesulfonyl)-isobutyl-amino]-6-(diethoxy-phosphoryloxy)-hexylcarbamoyl]-2,2-diphenyl-ethyl}-carbamic acid methyl ester (***XII***)

The product of step A above, (1*S*,5*S*)-(1-{5-[(4-amino-benzenesulfonyl)-isobutyl-amino]-6-phosphonooxy hexylcarbamoyl}-2,2-diphenyl-ethyl)-carbamic acid methyl ester (0.555 g, 0.73 mmol) was dissolved in 5 mL MeCN. Selectfluor (0.26 g, 0.7 mmol) was added and the mixture stirred for 30 min. The mixture was purified by reverse phase preparative HPLC and lyophilized to yield 278 mg (48% yield) white solid.

$^1$H NMR identical as previous entry, see first methodology above.

Step C. Preparation (1*S*,5*S*)-(1-{5-[(4-amino-3-fluoro-benzenesulfonyl)-isobutyl-amino]-6-phosphonooxy-hexylcarbamoyl}-2,2-diphenyl-ethyl)-carbamic acid methyl ester **(*XIII,*** in this specific case is compound ***XI***) (PL-515)

The procedure make this derivative was as described in the deprotection step for the methodology above. Yields 139 mg 70% after reverse phase HPLC.

$^1$H NMR identical as previous entry, see first methodology above.

**Example 11. Inhibitory activity and cytotoxicity**

The active ingredients which are released from the Lysine-based compounds described herein have been shown to inhibit HIV aspartyl protease (US patent no. 6,632,816). After oral dosing, plasma levels of the Lysine-based compound; PL-461 in rats are insignificant or under detection limits, compared to plasma levels of the active ingredient; PL-100, indicating that the phosphate moiety of the Lysine-based compound (e.g., PL-461) is metabolically cleaved and majority of this compound is converted to the corresponding active ingredient (e.g., PL-100) *in vivo.*

The cross-resistance profile of a representative active ingredient; PL-100 against 63 HIV isolates with reduced susceptibility to all approved protease inhibitors (PIs) including atazanavir is tested herein.

Briefly, the antiviral activity of PL-100 was determined using MT4 cells infected with a laboratory adapted HIV strain (NL4-3). For comparison, seven approved protease inhibitors (PIs) were tested in parallel. Result of this experiment is presented in Table 3.

These data suggest that the antiviral activity of PL-100 is comparable to that of the other PIs.

Cytotoxicity of PL-100 and other PIs was evaluated in the same cell culture system. The selectivity index (SI) is the ratio of cytotoxicity ($CC_{50}$) to antiviral activity ($EC_{50}$). The selective index is similar to the therapeutic index but relates to *in vitro* studies.

This result indicates that PL-100 is a potent, selective and non-cytotoxic PI.

Table 3

| Protease Inhibitor | $EC_{50}$ (nM) | $CC_{50}$ (nM) | SI ($CC_{50}$/$EC_{50}$) |
|---|---|---|---|
| **Atazanavir** | 4 | 55,000 | 13,750 |
| **Amprenavir** | 47 | > 100,000 | > 2,128 |
| **Indinavir** | 67 | > 100,000 | > 1,493 |
| **Lopinavir** | 19 | 28,000 | 1,474 |
| **Nelfinavir** | 29 | 8,000 | 276 |
| **Ritonavir** | 61 | 25,000 | 410 |
| **Saquinavir** | 12 | 19,000 | 1,583 |
| **PL-100** | 16 | 33,000 | 2,063 |

Atazanavir (ATV) is manufactured by BMS (Bristol Myers Squibb), Amprenavir (APV) is manufactured by GSK (Glaxo SmithKline), Indinavir (IDV) is manufactured by Merck, Lopinavir (LPV) is manufactured by Abott, Nelfinavir (NFV) is manufactured by Pfizer, Ritonavir (RTV) is manufactured by Abott, Saquinavir (SQV) is manufactured by Roche.

HIV PI class inhibitors may sometimes demonstrate binding to plasma protein which may affect their potency *in vivo.* The effect of 40% human serum on the antiviral activity of PL-100 in MT4 cells infected with NL4-3 was therefore evaluated. As shown in Table 4, the addition of human serum reduced the antiviral activity of PL-100 by 6-fold, which

result in an increase of its $EC_{50}$ to 100 nM. This is similar to what is reported for the approved drugs nelfinavir (NFV) and indinavir (IDV). So protein binding of PL-100 is not expected to be a significant issue in the clinic.

Table 4

| Culture condition | $EC_{50}$ nM (Fold Change) |
|---|---|
| 10% PBS* | 18 (1.0) |
| 10% FBS* + 40% human serum | 106 (5.9) |
| *Fetal bovine serum | |

**Example 12. Cross-resistance profile**

The cross-resistance profile of PL-100 against 63 HIV isolates with reduced susceptibility to all approved protease inhibitors (PIs) including atazanavir was thus tested.

The panel of 63 viral strains was selected based on the following rationale: 1) High-level loss of susceptibility to specific PIs; 2) High-level loss of susceptibility to multiple PIs; 3) Good representation of the primary mutations. This panel consists of resistant viruses from highly PI-experienced patients with high-level PI resistance.

The genotype of these viruses encompasses a wide variety of mutational patterns. We particularly looked for the following primary PI mutations: D30N, L33F/I, M46I/L, G48V, I50L/V, V82A/F/S/T, I84V and L90M as defined by the International AIDS Society (IAS)-USA. Table 5 illustrates the repartition and the strength of the chosen panel.

Table 5

| No. of primary PI mutations | No. of viral strains |
|---|---|
| 0 | 3 |
| 1 | 6 |
| 2 | 11 |
| 3 | 22 |
| 4 | 17 |
| 5 | 3 |
| 6 | 1 |

The mutation mentioned above refers to original (wild type) amino acid (one letter code)/position in the HIV-1 protease /resulting amino acid change(s) (one letter code). The one letter code amino acid is known in the art.

For example: D30N relates to : aspartic acid (D) found at position 30 of HIV-1 protease which has been changed (mutated) for an asparagine (N):

L33F/I relates to : leucine (L) found at position 33 of HIV-1 protease which has been changed (mutated) for either phenylalanine (F) or isoleucine (I); etc.

Table 6, illustrates the result of the cross-resistance profile of 63 diverse resistant strains

Table 6

| SAMPLE ID | Key Mutations[1] | Panel ID | Fold change in IC50 vs. Reference | | | | | | |
|---|---|---|---|---|---|---|---|---|---|
| | | | ATV | AMP | IDV | LPV | NFV | SQV | PL-100 |
| 03-129572 | 24I/33F/54V/82A/84V | 1 | | 13.2 | 10.4 | 43.4 | 31.4 | 85.4 | 4.2 |
| 03-129573 | 24I/33F/46I/54L/82A | 3 | | 7.7 | 3.3 | 16.9 | [illegible] | 1.5 | 8.4 |
| 03-129574 | 54V/82A/84V/90M | 5 | | 9.7 | 29.3 | 46.5 | 33.1 | 125.3 | 7 |
| 03-129575 | 33F/54V/73S/82A/84V/90M | 6 | | 68.8 | 32.8 | 130.1 | 172.1 | 400* | 37.2 |
| 03-129576 | 33F/46L/54V/82A/84V/90M | 7 | | 20.9 | 11.4 | 59.7 | 31.4 | 61.4 | 18.4 |
| 03-129578 | 46I/82T/84V | 11 | | 2.8 | 7.2 | [illegible] | 1.6 | 1 | 1.2 |
| 03-129579 | 46I/82T/84V | 13 | | 5.9 | 12.9 | 20.1 | 5.2 | 3.2 | 2.5 |
| 03-129580 | 46I/82T/84V/90M | 15 | | 4.5 | 31.4 | 17.2 | 39.9 | 105.7 | 4.8 |
| 03-129581 | 33I/46I/84V/88D/90M | 16 | | 8.7 | 38.5 | 28.3 | 71.7 | 192.8 | 6.3 |
| 03-129583 | 84V/90M | 21 | | 9.1 | 8.1 | [illegible] | 12.1 | 27 | 2.3 |
| 03-129584 | 33F/50V | 24 | | 36.8 | 1.3 | 12.8 | 3.6 | 1.7 | 2.7 |
| 03-129585 | 48V/54V/82A/90M | 26 | | 3.9 | 53.4 | 70.4 | 68.2 | 400* | 1.9 |
| 03-129586 | 30N/88D/90M | 28 | | 1.8 | 2.3 | 1.2 | 42.1 | 6 | 1.4 |
| 04-148709[2] | 32I/47A | 1 | 1 | 19.7 | 8.8 | 65.1 | 3.1 | 0.2 | 1.4 |
| 04-148710[2] | 46I/47V/84V | 118 | 7.1 | 20.1 | 3.6 | 19.5 | 5.1 | 11 | 4.3 |
| 04-148711 | 46I/47A/84V | 119 | 9.5 | 47.2 | 7.9 | 151.8 | 10.4 | 1.1 | 14.8 |
| 04-148712 | 46I/88S | 122 | 13.5 | 0.3 | 10.3 | 1.7 | 28.9 | 1.9 | 0.8 |
| 04-148713 | 46I/88S | 123 | 3.1 | 0.2 | 2 | 0.7 | 4.6 | 0.6 | 0.4 |
| 04-148714 | 46I/88S | 124 | 9.9 | 0.9 | 11.3 | 2.6 | 25.2 | 2 | 1.1 |
| 04-148715 | 33F/54L/88S/90M | 9 | 65 | 5.9 | 8 | [illegible] | 41.4 | 31.7 | 3.3 |
| 04-148716 | 33F/54L/88S/90M | 10 | 18.9 | 9.3 | [illegible] | [illegible] | 14.5 | 18.5 | 1.7 |
| 04-148718 | 33I/46I/84V/88D/90M | 19 | 16.4 | 5.7 | 26.2 | [illegible] | 39.6 | 133.9 | 5.6 |
| 04-148719 | 33F/46I/84V/88D/90M | 20 | 38 | 21 | 6.9 | 42.5 | 27.9 | 400* | 8.8 |
| 04-148720 | 30N/33F/46L/54L/84V/88D | 125 | 66.1 | 106.3 | 9.3 | 37.9 | 400* | 237.3 | 23.2 |
| 04-148721 | 32I/46I/47V/50L | 127 | 81.2 | 7.9 | 2.1 | [illegible] | 3.6 | 0.5 | 1.1 |
| 04-148722 | 33F/54L/82A/90M | 40 | 10.7 | 20.9 | 8.4 | [illegible] | 13.6 | [illegible] | 51.5 |
| 04-148723 | 33F/54L/82A/90M | 42 | [illegible] | 13.6 | 7.5 | 13.1 | 10.4 | 3.2 | 30.3 |
| 04-148724 | 33F/54V/73S/82A/90M | 44 | 11.9 | 8.4 | 18.5 | 52.9 | 20 | 16.7 | 5.3 |
| 04-148725 | 32I/46L/47V/84V | 53 | 25 | 89.4 | 21.7 | 133.9 | [illegible] | 0.8 | 0.6 |
| 04-148726 | 33F/54L/82A/84V | 132 | 44.5 | 54.5 | 6.2 | 44.5 | 17.2 | 37.6 | 31.2 |
| 04-148727 | 33F/54V/82A/84V | 133 | 5.5 | 37.9 | 18.7 | 119.6 | [illegible] | [illegible] | 3.4 |
| 04-148728 | 33F/46I/53L/82A/84V | 134 | 18.3 | 20.8 | [illegible] | 21.1 | 11.1 | 23.2 | 17.8 |
| 04-148729 | 33F/54V/82T/84V/90M | 61 | 14.1 | 7.5 | 26.8 | 19.2 | 14.9 | 51 | 3.4 |
| 04-148730 | 33F/46L/53L/54V/82A | 67 | 22.7 | 15.3 | 16.8 | 65.9 | 28.3 | 14 | 5.5 |
| 04-148731 | 46L/54M/82L/84V/90M | 69 | 52.3 | 116.8 | 42.5 | 73.7 | 43.8 | 50.3 | 84 |
| 04-148732 | 46L/54V/82A/90M | 74 | 11.8 | 9.2 | 5.9 | 17.2 | 14.1 | 41.5 | 3.2 |
| 04-148733 | 46I/54V/82A/84V | 76 | [illegible] | 9.9 | 8.2 | 45.1 | [illegible] | [illegible] | [illegible] |
| 04-148734 | 46L/54V/73C/84V/90M | 78 | 55.2 | 26.6 | 93.2 | 36.9 | 82.6 | 400* | 10.8 |
| 04-148735 | 46I/82T/84V/90M | 81 | 32 | 7.6 | 30.2 | 15.2 | 27.1 | 79.3 | 10.3 |
| 04-148736 | 46I/82A/84V/90M | 82 | [illegible] | 7.3 | [illegible] | [illegible] | 11.2 | [illegible] | 10.1 |
| 04-148737 | 54V/82A/84V/90M | 84 | 50.5 | 38.5 | 37.4 | 103.3 | 36.1 | 133.9 | 28.3 |
| 04-148738 | 33F/54V/82A/84V/90M | 86 | 24.7 | 18.6 | [illegible] | 33.6 | 21.1 | 85.5 | 9.8 |
| 04-148739 | 48V/54V/82A/90M | 138 | 28 | 1.1 | 23.1 | 24.4 | 25.4 | 400* | 1.1 |
| 04-148740 | 46L/48V/82A/84V/90M | 141 | 91.6 | 35.6 | 36.5 | 44.7 | 43.6 | 400* | 13.4 |
| 04-148741 | 48V/82A/84V/90M | 142 | 57.7 | 24 | 34 | 18.7 | 27.3 | 400* | 13.5 |
| 04-148742 | 46L/48V/82A/84V | 94 | 21.5 | 6.6 | 7.4 | [illegible] | [illegible] | 36 | 1.2 |
| 04-148743 | 46L/48V/82A/90M | 95 | 35.4 | 8.9 | 36.1 | [illegible] | 30.8 | 320.7 | 7.5 |
| 04-148744 | 46L/50L/54V/82A | 98 | 68.9 | 8.9 | [illegible] | 10.6 | 11.8 | 2.8 | 3.2 |
| 04-148745 | 46I/50L/54V/82A | 99 | 67.5 | 3.2 | 7.2 | [illegible] | 14 | 2.2 | [illegible] |
| 04-148746 | 33I/46I/84V/90M | 105 | 78.2 | 10.8 | 73.7 | 18.1 | 165.1 | 270.8 | [illegible] |
| 04-148747 | 33I/46I/84V/90M | 107 | [illegible] | 10.8 | 30.3 | [illegible] | 28.5 | 44.4 | 3.6 |
| 04-148748 | 37S/41K/70E | 115 | 1.4 | 0.7 | 1.2 | 1 | 1.6 | 1.2 | 0.9 |
| 04-148749 | 37T/41K/70E | 151 | 1.5 | 1.9 | 1.4 | 1.3 | 1.9 | 1.2 | 1.9 |
| 04-148750 | 46I/53L/82T/90M | 172 | 33.7 | 7.3 | 33.5 | 17.9 | 32.9 | 64.1 | 8.3 |
| 04-148751 | 54V/84V | 171 | [illegible] | 4.3 | [illegible] | [illegible] | 15 | 12.2 | 0.8 |
| 04-148752 | 54V/84V | 152 | 33.2 | 14.9 | 19.5 | 28.5 | 32.9 | 102.1 | 1.2 |

In Table 6 above, ([1]) indicates that mutations used in selecting viruses in at least one group; mixtures were ignored for this purpose, ([2]) indicates non-B subtype and (*) indicates 50% inhibition not reached at highest drug concentration tested and that an arbitrary FC value (400) was therefore assigned.

The key mutations listed in Table 6, only refers to the position in the HIV-1 protease which has been mutated and the resulting amino acid (one letter code) found in the mutated protease of the resistant strain. The original amino acid is not indicated.

For example, 33F/46L/54V/82A/84V/90M (panel ID 7 of Table 6) indicates that amino acid at position 33 has been mutated to phenylalanine (F), amino acid at position 46 has been mutated to leucine (L), amino acid at position 54 has been mutated to valine (V), amino acid at position 82 has been mutated to alanine (A), amino acid at position 84 has been mutated to valine (V) and amino acid at position 90 has been mutated to methionine (M). Similarly with the other resistant viral strains listed in Table 6.

The results presented in the above Table 6 indicates the fold-change (FC) in $IC_{50}$ vs the reference strain (NL-4.3). The dark cells indicate a FC ≤ 10, the grey cells indicate FC values of 2.5 ≤ FC < 10 and the white cells indicate a FC value of; 0 ≤ FC < 2.5. The results of cross-resistance profile are further illustrated in the graph of **Fig.1.**

Table 7 summarizes the phenotypic susceptibility results obtained against the 63 diverse, multi-PI-resistant strains mentioned above.

Table 7

| | **ATV** | **APV** | **IDV** | **LPV** | **NFV** | **SQV** | **PL-100** |
|---|---|---|---|---|---|---|---|
| **Median FC** | 15.6 | 9.7 | 8.1 | 17.9 | 15 | 23.2 | 3.6 |
| **Mean FC** | 25.7 | 18.5 | 16.5 | 31.3 | 31.7 | 85 | 8.7 |
| **% FC < 2.5** | 16 | 14 | 13 | 10 | 10 | 27 | 37 |
| **% FC < 10** | 38 | 52 | 54 | 37 | 27 | 40 | 76 |
| **% FC > 50** | 22 | 8 | 5 | 19 | 10 | 37 | 3 |

In the cross-resistance profiling studies against the 63 resistant strains mentioned herein, $EC_{95}$ of PL-100 was determined against each strain. The median $EC_{95}$ represents the concentration required to inhibit 95% of the viral replication of 50% diverse drug-resistant HIV-1 variants tested. Table 8 illustrates the percentage of resistant strains tested with protein-binding adjusted $EC_{95}$s ≤ 200 ng/ml. The resistant strains are grouped by the number of primary mutations they have.

Table 8

| **No. of primary PI mutations** | **% viral strains with $EC_{95}$s ≤ 200ng/ml** |
|---|---|
| 0 | 100 |
| 1 | 100 |
| 2 | 36 |
| 3 | 23 |
| 4 | 0 |
| 5-6 | 0 |

The $EC_{95}$ of PL-100 was determined against each of the 63 resistant strains indicated herein. Table 9 illustrates the percentage of resistant strains tested with protein-binding adjusted $EC_{95}$s ≤ 630 ng/ml. The resistant strains are grouped by the number of primary mutations they have.

Table 9

| **No. of primary PI mutations** | **% viral strains with $EC_{95}$s ≤ 630ng/ml** |
|---|---|
| 0 | 100 |
| 1 | 100 |
| 2 | 82 |
| 3 | 55 |
| 4 | 29 |

(continued)

| No. of primary PI mutations | % viral strains with $EC_{95}$s $\leq$ 630ng/ml |
|---|---|
| 5-6 | 0 |

Results presented herein indicate that PL-100 has a favourable cross-resistance profile against several HIV isolates.

**Example 13. Bioavailability of compounds**

In order to improve the chemical stability, aqueous solubility of the active ingredients, various Lysine-based compounds, such as PL-100 derivatives (PL-461, PL-462, etc.) were designed, synthesized and purified (Fig. 2, Table 10). The active ingredient has been shown to be efficient against an HIV-1 aspartyl protease (U.S. patent no. 6,632,816). Also as mentioned herein, the active ingredients present potent antiviral activity when tested on non-mutated HIV-1 viral strain (NL4.3 as the wild type virus) as well as several mutant strains.

Various PL-100 precursors were thus developed and tested. Examples of representative PL-100 precursors are illustrated in Table 10. PL-100 and PL-337 have been chosen herein as representative active ingredient among those disclosed in U.S. patent No. 6,632,816 to Stranix et al, for improving pharmacokinetics of these potent inhibitors.

The compounds listed in Table 10 were prepared by following scheme 1, 1A, 2, 3, 4 or 5; and more particularly as described in each example listed above. The numbers of the compounds listed in Table 10 (Ex. No.) corresponds to the example numbers presented above.

Table 10: Structures of exemplary lysine based compounds and active ingredients

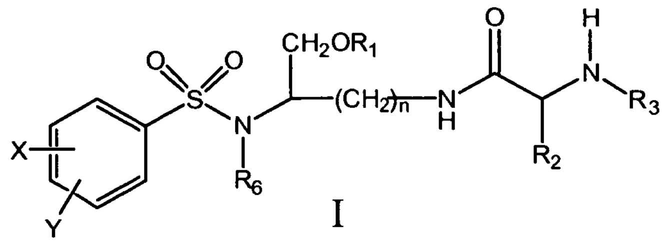

I

| Ex. No (PL-#) | X | Y | n | $R_1$ | $R_2$ | $R_3$ | $R_6$ | X'/Y' | D, L, DL *R, S, RS* |
|---|---|---|---|---|---|---|---|---|---|
| 1 (PL-461) | 4-$NH_2$ | H | 4 | $(HO)_2P(O)$ | $(C_6H_5)_2CH$ | $CH_3O$-CO | *iso*-butyl | H/H | *S*,*S* |
| 2 (PL-462) | 4-$NH_2$ | H | 4 | $(NaO)_2P(O)$ | $(C_6H_5)_2CH$ | $CH_3O$-CO | *iso*-butyl | H/H | *S*,*S* |
| 3 (PL-507) | 4-$NH_2$ | H | 4 | $(HO)_2P(O)$ | Naphthyl-2-$CH_2$ | $CH_3O$-CO | *iso*-butyl | H/H | |
| 4 (PL-498) | 4-$NH_2$ | H | 4 | $(HO)_2P(O)$ | Naphthyl-1-$CH_2$ | 4-morpholine-CO | *iso*-butyl | H/H | *S*,*S* |
| 5 (PL-504) | 4-$NH_2$ | H | 4 | $(HO)_2P(O)$ | $(C_6H_5)_2CH$ | $CH_3CO$ | *iso*-butyl | H/H | *S*,*S* |
| 6 (PL-515) | 4-$NH_2$ | 3-F | 4 | $(HO)_2P(O)$ | $(C_6H_5)_2CH$ | $CH_3O$-CO | *iso*-butyl | H/H | *S*,*S* |
| 11 (PL-100) | 4-$NH_2$ | H | 4 | H | $(C_6H_5)_2CH$ | $CH_3O$-CO | *iso*-butyl | H/H | |
| 12 (PL-337) | 4-$NH_2$ | 3-F | 4 | H | $C_6H_5)_2CH$ | $CH_3O$-CO | *iso*-butyl | H/H | |

To assess the extent of *in vivo* cleavage of the phosphate group from the putative compounds, PL-100, PL-462 (based on PL-100), PL-337 and PL-515 (based on PL-337) compounds were administered po (*per os,* (i.e., by mouth)) (50 mg/kg) to male Sprague-Dawley rats and their plasma concentration measured at different time intervals post-administration.

All test articles (PL-100, PL-462, PL-337 and PL-515) were prepared in different vehicle at the final concentration of 25 mg/mL. The vehicle composition is as follows: (1) 20% ethanol; 50% propylene glycol; 0.05% w/v Tween 20 and water (Mix); (2) PBS buffer (PBS).

Test articles were administered to male Sprague-Dawley rats at a single oral dose of 50 mg/kg. Each article was tested in three rats. Blood samples (0.2-0.3 mL) were collected at the post-dose time of 10, 20, 40, 60, 120, 180 and 360 minutes. The harvested blood was centrifuged to isolate plasma. The resulting plasma was separated and stored at -70°C.

As indicated above, when the Lysine-based compounds described herein are administered *in vivo,* the active ingredient is released and it may therefore inhibit the protease of retroviruses. For example, as illustrated in Fig.3 when PL-461 (or PL-462) is administered *in vivo*, the phosphate moiety (in the case of PL-461) is metabolically cleaved to generate the active ingredient; PL-100. Similarly when PL-515 is administered *in vivo*, the phosphate moiety is cleaved to generate PL-337.

Plasma samples together with standards and quality control samples were treated to precipitate proteins, then analyzed by HPLC-MS, for the presence of PL-462, PL-100, PL-515 and PL-337.

Table 11

| Compound | **PL-462** (Ex. No. 2) | **PL-100** (Ex. No. 1-F) | **PL-515** (Ex. No. 13) | **PL-337** (Ex. No. 13-A) |
|---|---|---|---|---|
| Vehicle | PBS | Mix | PBS | Mix |
| Number of rats | 3 | 3 | 3 | 3 |
| Dose (mg/Kg) | 50 po | 50 po | 50 po | 50 po |
| AUC (μg/hr*ml) | 0.816 ± 0.295 (PL-100, detected) | 0.675 ± 0.171 | 1.075± 0.625 (PL-337, detected) | 1.180 ± 0.196 |
| Cmax (nM) | 330 ± 109 | 498 ± 203 | 545± 215 | 681± 131 |
| Tmax (min) | 93± 60 | 40± 16 | 87± 60 | 60± 15 |
| In Table 11 above: 50 mg/Kg **PL-462** = 43 mg/Kg **PL-100**<br>50 mg/Kg **PL-515** = 43 mg/Kg **PL-337** | | | | |

The results demonstrate that PL-462 and PL-515 compounds may be delivered orally in aqueous solutions. None of the PL-462 and PL-515 compounds, delivered as aqueous solutions, are detected in the blood samples, which suggests rapid metabolism to PL-100 and PL-337 the parent drugs.

Aqueous dosing of PL-462 and PL-515 solutions showed equivalent to slightly superior delivery of PL-100 and PL-337 compared to non-aqueous formulations of PL-100 and PL-337.

Based on these results, all the phosphorylated compounds described in the present invention will demonstrate similar pharmacokinetic properties.

Partition coefficient (LogP) of selected compounds and the corresponding HIV protease inhibitors (drug) are as follow:

Table 12.

| **Compounds** | **LogP** | **Corresponding drugs** | **LogP** |
|---|---|---|---|
| PL-461 (or PL-462) | -1.2 | PL-100 | 3.6 |
| PL-515 | -0.75 | PL-337 | 3.8 |

The LogP were measured in a standard fashion by dissolving 1 mg of compound in 0.8 mL of each octanol and phosphate buffer pH 7.4 (0.04 M $KHPO_4$). The concentration of the compounds in the phases was detected by LC-MS. This test demonstrates the solubility of the compounds at physiological pH. The LogP obtained show that the compounds are highly soluble as compared to the corresponding drugs.

Results of solubility experiments illustrated at Fig. 4 indicates that PL-461 also possesses a moderate water

solubility in acidic conditions and that the solubility increases dramatically when pH >4.5. Water solubility of PL-100 and PL-461 at pH 7.5 is 0.079 and 145 mg/ml for PL-100 and PL-461, respectively.

The phosphorylated version of PL-100; namely PL-461, was selected for further evaluation due to its stability, solubility and oral bioavailability.

**Materials and Methods-pharmacokinetics studies**

Several studies were conducted in order to test the pharmacokinetic profile of the Lysine-based compounds described herein either alone or in combination with CYP450 inhibitors.

A first study **(study 1 #141690)** was conducted to evaluate the pharmacokinetic profile of PL-100 and PL-461 after a single dose oral administration in combination with ritonavir and two other studies **(study** 2 **#143656** and **study 3 #144536)** were conducted to evaluate the pharmacokinetic profile of PL-461 after a single dose oral administration in combination with ritonavir.

Animal tested are Sprague Dawley rats (species *Rattus norvegicus*) of 6-7 weeks old at the start of dosing and were obtained from Charles River (Montreal, Canada). The study usually comprises about 6 animals per groups.

Twenty four hours prior to the initiation of the study, rats were randomly selected into 6 groups, and each group had 6 rats (3 rats for each cage). The rats were fasted 3 h. before dosing and 3 h. after dosing.

The formulations are prepared as indicated below. The solutions are stable at room temperature over a week and are covered with aluminum foil to avoid lights. The mixture contains Ethanol, Propylene Glycol, Tween 20 and Water for Injection (20%, 50%, 0.1% and 30%, v/v/v/v). The pH of all solutions has been adjusted to 7.5. The dosing volume for all formulations is 2 mL per kg body weight.

Blood sample was collected at 15min, 30min, 45min, 1 h, 2h, 3h, 6h, 12h, 24h after the dosing. Blood samples were centrifuged to separate the plasma and separated plasma samples were placed into cryovials and frozen at -80°C for further analysis. Plasma samples together with standards and quality control samples were treated by protein precipitation and then analyzed by HPLC-MS.

**Protein precipitation**

Briefly, protein precipitation was performed on aliquot of 50.0 mL blank plasma (for standards and quality control samples (QCs)) or PK plasma. 5.00 μL of spiking solution was added for standards and QCs or acetonitrile / DMSO = 1/1 for PK samples.

The samples were mixed by vortexing for 10 seconds. 150 uL of internal standard working solution (1.00 ug/mL PL-459-01 in acetonitrile) was added. Samples were again vortexed for 10 seconds and sonicated for 5 mins. The samples were centrifuged for 10 mins at 10,000 rpm and 100 uL of the supernatant was transferred into an insert. 100 uL of deionized water (0.2% FA) was added and sample vortexed. Finally, 20 uL was injected in HPLC.

**Instrument Conditions for HPLC**

**HPLC:**

Column: Zorbax SB-C18, 2.1x50 mm, 5μm
Mobile phase: aqueous (Aq)=0.1 % formic acid in deionized water
Organic (Org)=0.1% formic acid in acetonitrile
Gradient: From 30% to 90% of Org in 5 mins, then return to 30% of Org in 1 min and equilibres 4 mins
Flow rate: 0.30 mL/min
Run time: 10 mins
Retention time: 5.9 min for PL-100 and PL-459 (IS), 4.6 min for PL-461, 6.3 min for ritonavir

**MSD:**

Source: ESI
Peakwidth : 0.07 min
Polarity: Positive

Acquisition time: 2.0 - 7.0 mins
Acquisition mode: SIM, MH+, 625.3 for PL-100; 630.3 for PL-459 (IS), 705.3 for PL-461, 721.3 for ritonavir
Fragmentor: 100
Gain : 1.5
Resolution: High
Spray chamber
Gas temperature: 325°C Drying gas: 12.0 L/min
Nebulizer pressure: 55 psig Vcap: 4000 V

**Bioavailability Calculations**

$$\text{Bioavailability of PL461} = \frac{(AUC_{0-24h} - p.o.)}{(AUC_{0-24h} - iv.)} \bullet \frac{Dose - iv}{Dose - po} \bullet \frac{M.W. - PL461}{M.W. - PL100} \bullet 100\%$$

$$\text{Bioavailability of PL100} = \frac{(AUC_{0-24h} - p.o.)}{(AUC_{0-24h} - iv.)} \bullet \frac{Dose - iv}{Dose - po} \bullet 100\%$$

Note:

Molecular Weight of PL461 = 705
Molecular Weight of PL100 = 624

**Test articles for studies 1, 2 and 3**

Unless specifically mentioned, female rats were used.

<u>Test articles of **study 1**</u>

| | |
|---|---|
| Name: | PL-100-06 |
| Batch / Lot No.: | 06 |
| Storage Conditions: | Room Temperature (15 - 30°C) |
| Name: | PL-461-05 |
| Batch / Lot No.: | 05 (ZL-177-091404) |
| Storage Conditions: | Room Temperature (15 - 30°C) |
| Name: | Ritonavir |
| Batch / Lot No.: | 749592E22 |
| Storage Conditions: | Room Temperature (15 - 30°C) |

<u>Formulation used in **study 1**</u>

Formulation 1:

PL-100-06 (30 mg/kg) concentration is 15mg/mL
PL-100-06; 90.0 mg dissolved in 6.00 mL Mix

Formulation 2:

PL-100-06 (30 mg/kg) concentration is 15mg/mL and Ritonavir (10 mg/kg) concentration is 5mg/mL
PL-100-06; 90.0 mg + 30 mg RTV dissolved in 6.00 mL Mix

Formulation 3:

PL-100-06 (30 mg/kg) concentration is 15mg/mL and Ritonavir (30 mg/kg) concentration is 15mg/mL
PL-100-06; 90.0 mg + 90 mg RTV dissolved in 6.00 mL Mix

Formulation 4:

PL-461-05 (30 mg/kg) concentration is 15mg/mL
PL-461-05; 90.0 mg dissolved in 6.00 mL Mix, pH 7.0

Formulation 5:

PL-461-05 (30 mg/kg) concentration is 15mg/mL and Ritonavir (10 mg/kg) concentration is 5mg/mL
PL-461-05; 90.0 mg + 30 mg RTV dissolved in 6.00 mL Mix, pH 7.0

Formulation 6:

PL-461-05 (30 mg/kg) concentration is 15mg/mL and Ritonavir (30 mg/kg) concentration is 15mg/mL
PL-461-05; 90.0 mg + 90 mg RTV dissolved in 6.00 mL Mix, pH 7.0

<u>Test articles of **study 2**</u>

| | |
|---|---|
| Name: | PL-461-05 |
| Batch / Lot No.: | 05 (ZL-7-177-091404) |
| Storage Conditions: | Room Temperature (15 - 30°C) |
| | |
| Name: | Ritonavir |
| Batch / Lot No.: | 749592E22 |
| Storage Conditions: | Room Temperature (15 - 30°C) |

<u>Formulation used in **study 2**</u>

Formulation 1:

PL-461-05 (29.93 mg/kg) concentration is 14.97mg/mL
PL-461-05; 89.8 mg dissolved in 6.00 mL Mix

Formulation 2:

PL-461-05 (30.2 mg/kg) concentration is 15.10mg/mL and Ritonavir (10 mg/kg) concentration is 5mg/mL
PL-461-05; 90.6 mg + 30 mg RTV dissolved in 6.00 mL Mix

Formulation 3:

PL-461-05 (29.43 mg/kg) concentration is 14.72mg/mL and Ritonavir (5.10 mg/kg) concentration is 2.55mg/mL
PL-461-05; 88.3 mg + 15.3 mg RTV dissolved in 6.00 mL Mix

Formulation 4:

PL-461-05 (51.27 mg/kg) concentration is 25.63mg/mL
PL-461-05; 153.8 mg dissolved in 6.00 mL Mix, pH 7.0

Formulation 5:

PL-461-05 (50.03 mg/kg) concentration is 25.02mg/mL and Ritonavir (17.03 mg/kg) concentration is 8.52mg/mL
PL-461-05; 150.1 mg + 51.1 mg RTV dissolved in 6.00 mL Mix, pH 7.0

Formulation 6:

PL-461-05 (49.33 mg/kg) concentration is 24.67mg/mL and Ritonavir (8.03 mg/kg) concentration is 4.02mg/mL
PL-461-05; 148.0 mg + 24.1 mg RTV dissolved in 6.00 mL Mix, pH 7.0

<u>Test articles of **study 3**</u>

| | |
|---|---|
| Name: | PL-461-06 |
| Batch / Lot No.: | 06 (SAP-8-57-102604) |
| Storage Conditions: | Room Temperature (15 - 30°C) |
| Name: | Ritonavir (RTV) |
| Batch / Lot No.: | 749592E22 |
| Storage Conditions: | Room Temperature (15 - 30°C) |

<u>Formulation used in **study 3**</u>

Formulation 1:

PL-461-06 (30 mg/mL) dose is 60mg/kg
PL-461-06; 180.0 mg dissolved in 6.00 mL Mix

Formulation 2:

PL-461-06 (15 mg/mL) dose is 30mg/kg and Ritonavir (5 mg/mL) dose is 10mg/kg
PL-461-06; 90.0 mg + 30 mg RTV dissolved in 6.00 mL Mix

Formulation 3:

PL-461-06 (50 mg/mL) dose is 100mg/kg and Ritonavir (5 mg/mL) dose is 10mg/kg
PL-461-06; 300.0 mg + 30 mg RTV dissolved in 6.00 mL Mix

Formulation 4:

PL-461-06 (50 mg/mL) dose is 100mg/kg
PL-461-06; 300.0 mg dissolved in 6.00 mL Mix

Formulation 5:

PL-461-06 (30 mg/mL) dose is 60mg/kg and Ritonavir (5 mg/mL) dose is 10mg/kg
PL-461-06; 180.0 mg + 30 mg RTV dissolved in 6.00 mL Mix

Formulation 6:

PL-461-06 (50 mg/mL) dose is 100mg/kg and Ritonavir (8.33 mg/mL) dose is 16.67mg/kg
PL-461-06; 300 mg + 50 mg RTV dissolved in 6.00 mL Mix

Test articles of **study 4**

PL-461-06
Batch / Lot No.: 06 (SAP-8-57-102604)
Storage Conditions: Room Temperature (15 - 30°C)

Ritonavir (RTV)
Batch / Lot No.: 21-802AW21
Storage Conditions: Room Temperature (15 - 30°C)
Manufacturer: Abbott Laboratories, Limited

Preparation used in **study 4**

**PL-461-06 Preparation 1**

| | |
|---|---|
| Test Articles: | PL-461 (20 mg/mL) |
| Vehicle: | Water for injection |
| pH: | 6.0 |
| Appearance: | Pale yellow solution |
| Storage Condition: | Room temperature (15-30°C) |

**PL-461-06 Preparation 2**

| | |
|---|---|
| Test Articles: | PL-461 (60 mg/mL) |
| Vehicle: | Water for injection |
| pH: | 6.0 |

(continued)

| **PL-461-06 Preparation 2** | |
|---|---|
| Appearance: | Pale yellow solution |
| Storage Condition: | Room temperature (15-30°C) |
| **PL-461-06 Preparation 3** | |
| Test Articles: | PL-461 (200 mg/mL) |
| Vehicle: | Water for injection |
| pH: | 6.0 |
| Appearance: | Yellow solution |
| Storage Condition: | Room temperature (15-30°C) |

Ritonavir Solution (16 mg/mL)

2 mL of the Ritonavir Solution, Norvir, (80 mg/mL) is diluted with 8 mL mixture containing 60% Propylene Glycol and 40% Water. The final concentration of Ritonavir is 16.0 mg/mL.

**Example 14. Pharmacokinetics of PL-461**

The pharmacokinetics (PK) of PL-461 was tested and compared with that of the original active ingredient.

The PK profile was obtained by administration of the test drugs; PL-100 and PL-461 in rats. PL-100 or PL-461 was administered orally at doses indicated in Fig. 5. Each time point in the figure represents the average plasma [PL-100] (ng/ml) of 6 female rats at a given dose.

The results indicate that the absolute oral bioavailability for PL-100 and PL-461 at 30 mg/kg is 8.7 and 23%, respectively. The bioavailability of PL-461 at 100mg/kg is 23%.

The horizontal line in Fig. 5 represents plasma [PL-100] of 200 ng/ml. The PK profile of PL-461 shows that the time (t) > 200 ng/ml is approximately 6 hours in rats at 100 mg/kg, suggesting PL-461 has a potential as a twice daily drug to maintain such plasma [PL-100] in man at an equivalent dose.

The pharmacokinetic parameters of PL-100 and PL-461 in rats** was compared. These results are summarized in Table 13 below.

Table 13

| | Dose mg/kg | Route | λz (1/hr) | T1/2 (hr) | Tmax (hr) | Cmax (ng/ml) | Tlast (hr) | Clast (ng/ml) | AUClast (hr ng/ml) | AUCinf (hr ng/ml) | Vz_F (L/kg) | Cl_F (L/hr/kg) |
|---|---|---|---|---|---|---|---|---|---|---|---|---|
| PL-100* | 5 | i.v. | 0.52 | 1.2 | 0.1 | 1804 | 6 | 44 | 1369 | 1434 | 6.1 | 3.5 |
| PL-100* | 50 | p.o. | 0.09 | 8.4 | 0.4 | 457 | 24 | 15 | 1101 | 1284 | 549 | 44 |
| PL-461* | 50 | p.o. | 0.10 | 9.4 | 0.5 | 1216 | 24 | 13 | 2226 | 2416 | 282 | 23 |

In Table 13, the abbreviations are as follows:
*λz elimination rate constant*
*t1/2 plasma elimination half-life*
*Tmax time of maximum concentration*
*Cmax maximum concentration*
*Tlast time of last measurable concentration*
*Clast last measurable concentration*
*AUClast area under the plasma conc-time curve from zero to the last measurable concentration*
*AUCinf area under the plasma concentration-time curve from zero extrapolated to infinity*
*Vz_F apparent volume of distribution*
*Cl_F apparent oral clearance*
*Each treatment group had at least 6 female rats.
** PK analysis was done using the non-compartmental method with WinNonLin Professional (version 4.0). AUC was calculated using the linear-up/log-down method.

Results presented in Fig. 5 and in Table 13 indicate that PL-461 has improved PK over PL-100.

**Example 15. Methods and compositions for improving the pharmacokinetics of active ingredients and Lysine-based compounds**

The results presented herein indicate that although the active ingredients (e.g., PL-100, etc.) and the Lysine-based compounds (e.g., PL-461, PL-462, etc.) are good anti-viral compounds, the pharmacokinetics may still be improved.

Further experiments were therefore conducted to that effect by administering PL-100 or PL-461 with a drug able to inhibit cytochrome P450 monooxygenase (CYP450) (i.e., a CYP450 inhibitor) in order to test whether this type of inhibitor may increase the pharmacokinetics of compounds described herein.

Ritonavir, quinidine, ketoconazole and sulfaphenazole were chosen among candidate CYP450 inhibitors and the pharmacokinetics of PL-461 and CYP450 inhibitors combinations was compared with the pharmacokinetics of other PI and CYP450 inhibitors combination. The effect of CYP450 inhibitors on the metabolism of HIV-1 anti-protease was tested in human liver microsomes.

Although, ketoconazole and sulfaphenazole are able to increase the PK of PL-100, results of Fig. 6 indicate that the most effective combination is that of PL-100 and ritonavir.

Results presented in Fig. 7 and in Table 14 illustrate the bioavailability of PL-100 or PL-461 when administered in combination with a CYP450 inhibitor.

**Table 14. Oral bioavailability of PL-100 and PL-461 in female rats (# 141690)**

| Group No. | Test article | Dose* (mg/Kg) | Dose** (mg/Kg) | Absolute Bioavailability*** (%) | | | | | | Ave. | SD | CV(%) | Max | Min | Median |
|---|---|---|---|---|---|---|---|---|---|---|---|---|---|---|---|
| 1 | | 30/0 | 30/0 | 8.5 | 8.4 | 8.5 | 9.0 | 7.2 | 10.7 | **8.7** | 1.1 | 12.8 | 10.7 | 7.2 | **8.5** |
| 2 | PL100/RTV | 30/10 | 33/10 | 36.3 | 25.0 | 24.8 | 31.3 | 21.9 | 33.7 | **28.8** | 6.3 | 19.9 | 36.3 | 21.9 | **28.1** |
| 3 | | 30/30 | 32/30 | 70.0 | 54.6 | 75.3 | 54.8 | 70.2 | 41.2 | **61.0** | 13.0 | 21.3 | 75.3 | 41.2 | **62.4** |
| 4 | | 30/0 | 35/0 | 15.9 | 33.6 | 13.0 | 14.9 | 22.3 | 23.8 | **20.6** | 7.7 | 37.3 | 33.6 | 13.0 | **19.1** |
| 5 | PL461/RTV | 30/10 | 34/11 | 80.7 | 45.1 | 105.1 | 94.3 | 47.7 | 36.5 | **68.2** | 28.8 | 42.3 | 105.1 | 36.5 | **64.2** |
| 6 | | 30/30 | 34/34 | 32.7 | 93.8 | 42.1 | 64.9 | 66.7 | 43.5 | **57.3** | 22.4 | 39.1 | 93.8 | 32.7 | **54.2** |

***: Intended dose by protocol**
****: Corrected dose by QC results.**
*****: Bioavailability was calculated from corrected dose.**

These results indicate that ritonavir (RTV: a CYP450 inhibitor) increases the bioavailability of PL-100 and that of PL-461 as well.

Further CYP450 inhibitor efficient at increasing the pharmacokinetics of the active ingredients or the Lysine-based compounds (referred here as "compounds") described herein may be identified by co-administering a putative CYP450 inhibitor and a desired compound (one or more compounds) in an assay (e.g., microsome assay or *in vivo* studies) described herein and to measure the amount of residual compound (active ingredient) still remaining after various time points following administration (incubation). A CYP450 inhibitor which increases the bioavailability of the compound (active ingredient) compared to the bioavailability measured without the putative CYP450 inhibitor would be found efficient for the purpose of improving the pharmacokinetics of the compound.

In study 4, ritonavir was first administered and PL-1461 was administered 15 or 30 minutes later. The result obtained using this administration scheme (Table 15) indicates that this method is as efficient at increasing the pharmacokinetics of the Lysine-based compound disclosed herein as the method of studies 1-3.

Table 15

| Group | Test Article | Dose (mg/kg) | Conc. of Test Article (mg/mL) | Route of Administration | Dosing Volume (mL/kg) | Number of Animals per Group |
|---|---|---|---|---|---|---|
| **1** | PL-461-06 | 100 | 20 | Oral | 5 | 3 male / 3 female |
| **2** | PL-461-06 | 300 | 60 | Oral | 5 | 3 male / 3 female |
| **3** | PL-461-06 | 1000 | 200 | Oral | 5 | 3 male / 3 female |
| **4** | RTV | 16 | 16 | Oral | 1 | 3 male / 3 female |
| | PL-461-06 | 100 | 20 | Oral | 5 | |
| **5** | RTV | 16 | 16 | Oral | 1 | 3 male / 3 female |
| | PL-461-06 | 100 | 20 | Oral | 5 | |
| **6** | RTV | 16 | 16 | Oral | 1 | 3 male / 3 female |
| | PL-461-06 | 100 | 20 | Oral | 5 | |

**Example 16. Pharmacokinetics of Lysine-based compounds and CYP450 inhibitors combination**

In studies 1, 2 and 3 mentioned above, PL-461 and RTV were co-administered orally at doses indicated in Fig. 8.

Each time point in Fig. 8 represents the average plasma concentration of PL-100 (ng/ml) of 6 female rats at a given dose. The horizontal line in Fig.8 represents plasma concentration of PL-100 of 630 ng/ml. The PK profile shows that the time (t) > 630 ng/ml is approximately 6 hours in rats at 100 mg/kg PL-461 and 16.7 mg/kg RTV, suggesting that PL-461 has a potential as a twice daily drug to maintain such plasma concentration of PL-100 in man at an equivalent dose.

**Example 17. Optimization of Lysine-based compounds *vs* CYP450 inhibitor ratio**

The absolute oral bioavailability of PL-461 was determined under various conditions as indicated in Fig.9. More particularly, the concentration of PL-461 and the proportion of PL-461 compared to CYP450 was varied. Results presented herein indicate that sufficient oral bioavailability, relative to protein binding adjusted $EC_{95}$ against resistant strains may be achieved when boosted at a ratio of 6 (PL-461) to 1 (RTV), although other ratios may also successfully be used as indicated in Table 16 below.

**Table 16. Oral bioavailability of PL-461 in rats with and without ritonavir (#141690, #143656, #144536)**

| Dose level of PL461 | Ratio of dose (PL461/RTV) | Dose* (mg/kg) (PL461/RTV) | Absolute Bioavailability (%)** | | | | | | Ave. | SD | CV(%) | Max | Min | Median |
|---|---|---|---|---|---|---|---|---|---|---|---|---|---|---|
| 30 mg/kg | N/A*** | 35/0 | 15.9 | 33.6 | 13.0 | 14.9 | 22.3 | 23.8 | **22.5** | 6.9 | 30.7 | 33.6 | 13.0 | **23.8** |
| | | 27/0 | 30.1 | 24.0 | | 24.5 | | | | | | | | |
| | 6/1 | 26/3.5 | 42.2 | 37.6 | | 45.1 | 65.5 | | **47.4** | 12.3 | 26.0 | 65.5 | 37.6 | **43.7** |
| | 3/1 | 34/11 | 80.7 | 45.1 | 105.1 | 94.3 | 47.7 | 36.5 | **52.6** | 24.5 | 46.6 | 105.1 | 26.5 | **43.9** |
| | | 26/9.2 | 62.1 | 45.3 | 26.5 | 38.5 | 42.6 | 34.7 | | | | | | |
| | | 26/8.4 | 34.7 | 37.1 | 32.9 | 28.6 | 54.9 | 39.4 | | | | | | |
| | 1/1 | 34/34 | 32.7 | 93.8 | 42.1 | 64.9 | 66.7 | 43.5 | **57.3** | 22.4 | 39.1 | 93.8 | 32.7 | **54.2** |
| 50 mg/kg | N/A*** | 48/0 | 11.1 | 20.3 | 3.2 | 23.3 | | 31.3 | **17.4** | 7.7 | 44.6 | 31.3 | 7.1 | **14.7** |
| | | 51/0 | 12.0 | 7.1 | 14.7 | 24.4 | 13.4 | 24.4 | | | | | | |
| | 6/1 | 55/11 | 34.9 | 51.7 | 33.9 | 58.4 | 46.0 | 24.1 | **41.5** | 12.8 | 30.7 | 58.4 | 24.1 | **40.5** |
| | 3/1 | 45/15 | 49.0 | 64.5 | 41.9 | 42.3 | 13.8 | 85.2 | **49.5** | 24.0 | 48.6 | 85.2 | 13.8 | **45.7** |
| 100 mg/Kg | N/A*** | 84/0 | 13.6 | 37.1 | 25.3 | 25.6 | 15.2 | | **23.4** | 9.5 | 40.6 | 37.1 | 13.6 | **25.3** |
| | 10/1 | 85/9.2 | 25.2 | 20.1 | 40.0 | 20.3 | 28.9 | 51.4 | **31.0** | 12.4 | 40.0 | 51.4 | 20.1 | **27.1** |
| | 6/1 | 89/17 | 91.2 | 47.0 | 39.1 | 56.7 | 35.6 | | **53.9** | 22.4 | 41.5 | 91.2 | 35.6 | **47.0** |

*: **Corrected dose by QC results.**

***Table 10: Structures of exemplary lysine based compounds and active ingredients***: **Bioavailability was calculated by corrected dose.**

*****: No Ritonavir**

Finally Fig. 10 indicates that PL-100 inhibits CYP3A4/5 as does RTV. RTV's higher Ki (about 10-fold higher) confirms that it is able to act as a boosting agent for PL-100.

It was shown herein that PL-100 is a potent, specific and non-cytotoxic novel PI and that it has a favorable cross-resistance pattern compared to all approved PIs.

PL-461, a precursor of PL-100, is >1800-fold more water soluble than PL-100 and has a 2 to 3-fold improved oral bioavailability over PL-100. PL-461 has a potential as a novel PI for the treatment of patients infected with PI-resistant HIV strains bearing, for example, two or less primary mutations. PL-461, when combined with a CYP450 inhibitor such as, for example, ritonavir, has a great potential as a novel PI for the treatment of patients infected with PI-resistant HIV strains bearing mutations (e.g., two or more primary mutations). The ratio of PL-461 to ritonavir required for boosting has been found herein adequate for *in vivo* administration.

On the basis of PL-100 cross-resistance data and the pharmacokinetics results, it is expected that equivalent doses of PL-461 in man will effectively inhibit replication of protease-resistant HIV strains. Moreover, these data also suggests that PL-461 may be administered, for example, using a convenient twice-daily dosing regimen.

## Claims

**1.** A pharmaceutical composition comprising:

a) a lysine-based compound of formula II

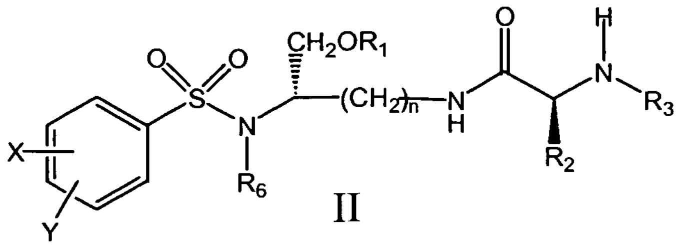

II

or a pharmaceutically acceptable salt thereof, wherein:

n is 4,
X is 4-$NH_2$,
YisH
$R_6$ is *iso*-butyl,
$R_3$ is $CH_3O$-CO-,
X' and Y' are H,
$R_2$ is a diphenylmethyl group of formula IV

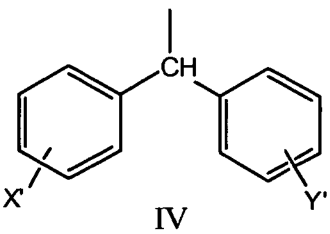

IV

,

$R_1$ is $(HO)_2P(O)$;

b) ritonavir; and
c) a pharmaceutically acceptable carrier;

wherein the ratio (w/w) of said lysine-based compound, or pharmaceutically acceptable salt thereof, to ritonavir is 6:1 to 3:1.

**2.** A pharmaceutical combination comprising:

a) a lysine-based compound of formula II

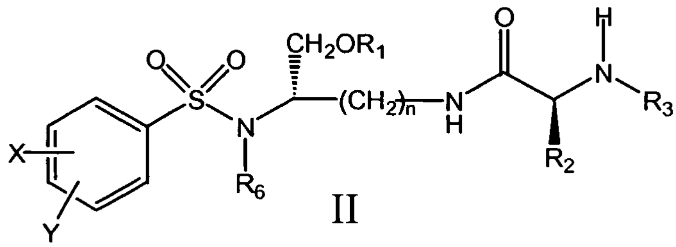

II

or a pharmaceutically acceptable salt thereof, wherein:

n is 4,
X is 4-$NH_2$,
YisH
$R_6$ is *iso*-butyl,
$R_3$ is $CH_3O$-CO-,
X' and Y' are H,
$R_2$ is a diphenylmethyl group of formula IV

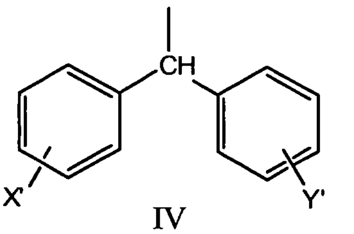

IV

,

$R_1$ is $(HO)_2P(O)$; and

b) ritonavir;

wherein the ratio (w/w) of said lysine-based compound, or pharmaceutically acceptable salt thereof, to ritonavir is 6:1 to 3:1.

**3.** The pharmaceutical combination of claim 2, wherein the lysine based compound of formula II and ritonavir are formulated as separate compositions which may be administered separately at the same time or at different times.

**4.** The use of the lysine-based compound of formula II

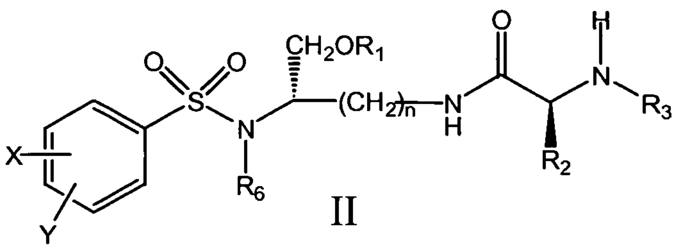

II

or a pharmaceutically acceptable salt thereof and ritonavir in the manufacture of a pharmaceutical composition for the treatment or prevention of an HIV infection or for treatment or prevention of AIDS,
wherein n, X, Y, X', Y', $R_1$, $R_2$, $R_3$, and $R_6$ are as defined in claim 1;

and wherein the ratio (w/w) of the lysine-based compound and the ritonavir is 6:1 to 3:1.

**5.** A pharmaceutical composition as defined in claim 1, or a pharmaceutical combination as defined in claim 2 or 3, for use in treating or preventing an HIV infection or treating or preventing AIDS.

**6.** The lysine based compound of formula II

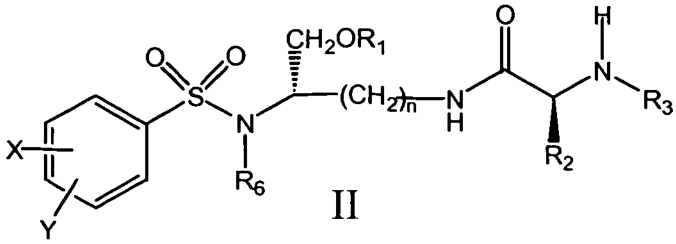

II

wherein n, X, Y, X', Y', $R_1$, $R_2$, $R_3$, and $R_6$ are as defined in claim 1 or a pharmaceutically acceptable salt thereof, and ritonavir for use in treating or preventing an HIV infection or treating or preventing AIDS wherein ritonavir is in an amount which is sufficient to reduce the metabolism of the lysine based compound of formula II and wherein the ratio (w/w) of said lysine based compound to ritonavir is 6:1 to 3:1.

**Patentansprüche**

**1.** Pharmazeutische Zusammensetzung, umfassend:

a) eine auf Lysin basierende Verbindung der Formel II

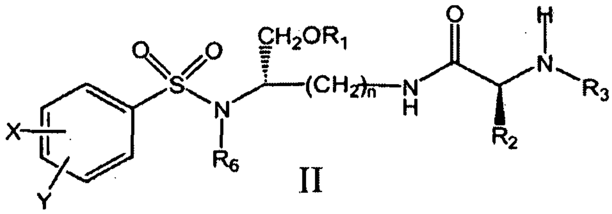

II

oder ein pharmazeutisch annehmbares Salz davon, wobei:

n 4 ist,
X 4-$NH_2$ ist,
Y H ist,
$R_6$ *iso*-Butyl ist,
$R_3$ $CH_3O$-CO- ist,
X' und Y' H sind,
$R_2$ eine Diphenylmethylgruppe der Formel IV ist

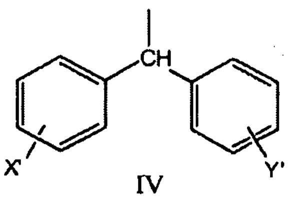

IV

,

$R_1$ $(HO)_2P(O)$ ist;

b) Ritonavir; und

c) einen pharmazeutisch annehmbaren Träger;

wobei das Verhältnis (w/w) der auf Lysin basierenden Verbindung, oder des pharmazeutisch wirksamen Salzes davon, zu Ritonavir 6:1 bis 3:1 beträgt.

**2.** Pharmazeutische Kombination, umfassend:

a) eine auf Lysin basierende Verbindung der Formel II

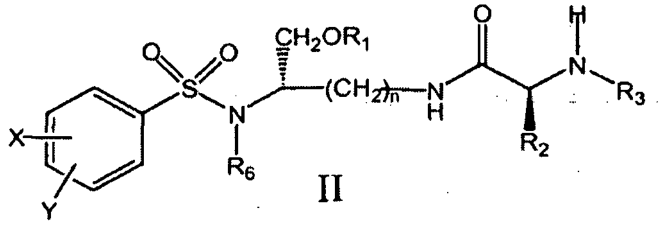

II

oder ein pharmazeutisch annehmbares Salz davon, wobei:

n 4 ist,
X 4-$NH_2$ ist,
Y H ist,
$R_6$ *iso*-Butyl ist,
$R_3$ $CH_3O$-CO- ist,
X' und Y' H sind,
$R_2$ eine Diphenylmethylgruppe der Formel IV ist

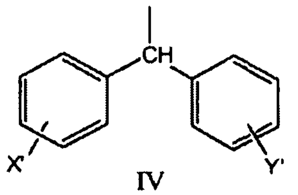

IV

,

$R_1$ $(HO)_2P(O)$ ist; und

b) Ritonavir;

wobei das Verhältnis (w/w) der auf Lysin basierenden Verbindung, oder des pharmazeutisch wirksamen Salzes davon, zu Ritonavir 6:1 bis 3:1 beträgt.

**3.** Pharmazeutische Kombination nach Anspruch 2, wobei die auf Lysin basierende Verbindung der Formel II und Ritonavir als separate Zusammensetzungen formuliert sind, die separat gleichzeitig oder zu unterschiedlichen Zeiten verabreicht werden können.

**4.** Verwendung der auf Lysin basierenden Verbindung der Formel II

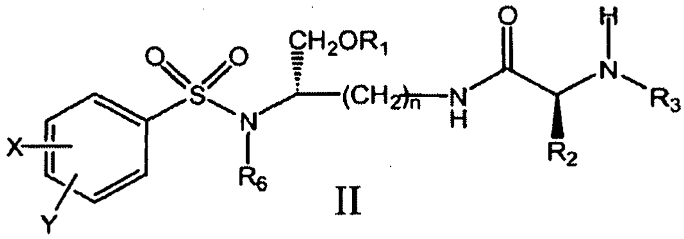

II

oder ein pharmazeutisch annehmbares Salz davon und Ritonavir bei der Herstellung einer pharmazeutischen Zusammensetzung für die Behandlung oder Prävention einer HIV-Infektion oder zur Behandlung oder Prävention von AIDS,
wobei n, X, Y, X', Y', $R_1$, $R_2$, $R_3$, und $R_6$ wie in Anspruch 1 definiert sind;
und wobei das Verhältnis (w/w) der auf Lysin basierenden Verbindung zu dem Ritonavir 6:1 bis 3:1 beträgt.

**5.** Pharmazeutische Zusammensetzung wie in Anspruch 1 definiert, oder eine pharmazeutische Kombination wie in Anspruch 2 oder 3 definiert, zur Verwendung bei der Behandlung oder Prävention einer HIV-Infektion oder Behandlung oder Prävention von AIDS.

**6.** Auf Lysin basierende Verbindung der Formel II

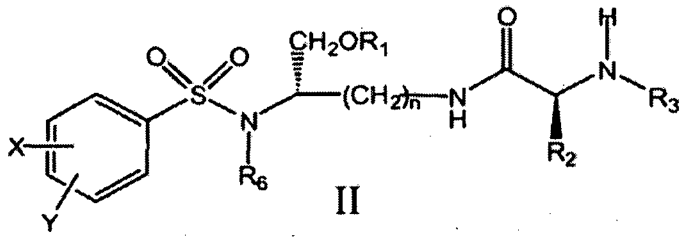

II

wobei n, X, Y, X', Y', $R_1$, $R_2$, $R_3$, und $R_6$ wie in Anspruch 1 definiert oder ein pharmazeutisch annehmbares Salz davon sind, und
Ritonavir zur Verwendung bei der Behandlung oder Prävention einer HIV-Infektion oder Behandlung oder Prävention von AIDS, wobei Ritonavir in einer Menge vorliegt, die ausreicht, um den Metabolismus der auf Lysin basierenden Verbindung der Formel II zu verringern, und wobei das Verhältnis (w/w) der auf Lysin basierenden Verbindung zu Ritonavir 6:1 bis 3:1 beträgt.

## Revendications

**1.** Une composition pharmaceutique comprenant :

a) un composé à base de lysine de la formule II

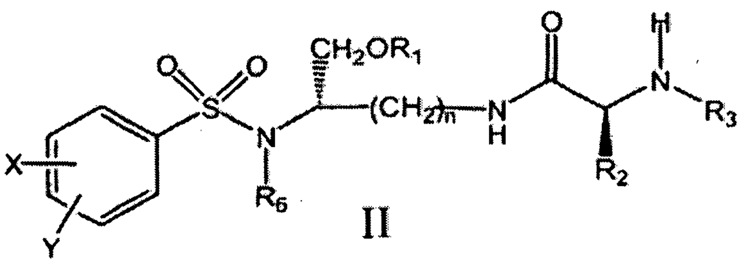

II

ou un sel dérivé, acceptable dans la pharmacie, où :

n est 4,
X est 4-$NH_2$,

Y est H,
$R_6$ est de l'*iso*-butyl,
$R_3$ est du $CH_3O$-CO-,
X' et Y' sont H,
$R_2$ est un groupe de diphénylméthyle de la formule IV

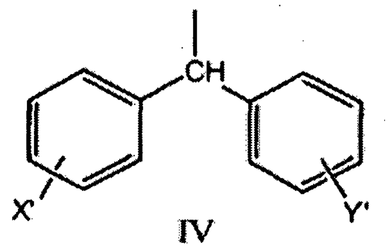

$R_1$ est $(HO)_2P(O)$ ;

b) ritonavir; et
c) un porteur acceptable dans la pharmacie ;

où le ratio (w/w) dudit composé à base de lysine, ou le sel dérivé acceptable dans la pharmacie, par rapport au ritonavir est de 6:1 à 3:1

**2.** Une combinaison pharmaceutique comprenant :

a) un composé à base de lysine de la formule II

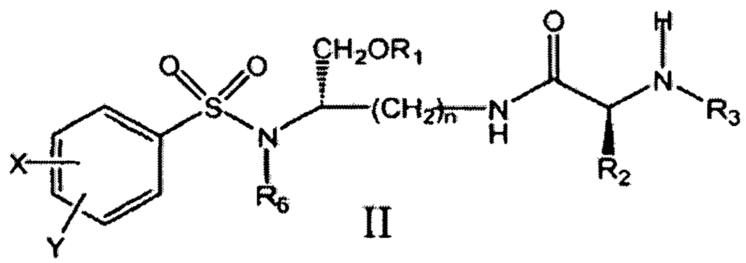

ou un sel dérivé acceptable dans la pharmacie, où :

n est 4,
X est 4-$NH_2$,
Y est H,
$R_6$ est de l'*iso*-butyl,
$R_3$ est du $CH_3O$-CO-,
X' et Y' sont H,
$R_2$ est un groupe de diphénylméthyle de la formule IV

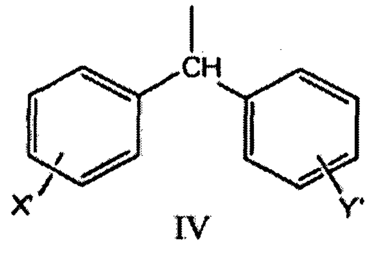

$R_1$ est $(HO)_2P(O)$ ; et

b) ritonavir ;

où le ratio (w/w) dudit composé à base de lysine, ou le sel dérivé acceptable dans la pharmacie, par rapport au ritonavir est de 6:1 à 3:1

**3.** La combinaison pharmaceutique de la revendication 2, où le composé à base de lysine de la formule II et le ritonavir sont formulés comme des compositions distinctes qui peuvent être administrées séparément, simultanément ou à des moments différents.

**4.** L'utilisation d'un composé à base de lysine de la formule II

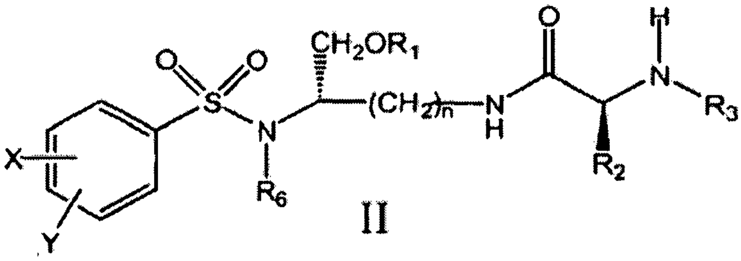

II

ou un sel dérivé acceptable dans la pharmacie et du ritonavir fabriqué sous la forme d'une composition pharmaceutique pour le traitement ou la prévention d'une infection par le VIH ou pour le traitement ou la prévention du SIDA, Où n, X, Y, X', Y', $R_1$, $R_2$, $R_3$ et $R_6$ sont définis dans la revendication 1 ;
et où le ratio (w/w) du composant à base de lysine et le ritonavir est de 6:1 à 3:1.

**5.** Une composition pharmaceutique telle que définie dans la revendication 1, ou une combinaison pharmaceutique telle que définie dans la revendication 2 ou 3, destinée au traitement ou à la prévention d'une infection par le VIH ou le traitement ou la prévention du SIDA.

**6.** Le composé à base de lysine de la formule II.

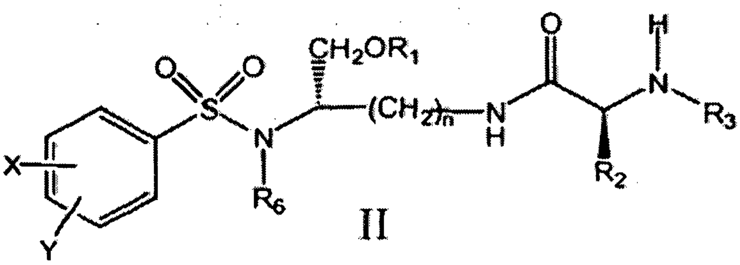

II

Où n, X, Y, X', Y', $R_1$, $R_2$, $R_3$ et $R_6$ sont conformes à la définition donnée dans la revendication 1 ou un sel dérivé acceptable dans la pharmacie, et
Ritonavir utilisé pour le traitement ou la prévention d'une infection par le VIH ou le traitement ou la prévention du SIDA, où le ritonavir est présent dans une quantité suffisante pour réduire le métabolisme du composé à base de lysine de la formule II et où le ratio (w/w) dudit composé à base de lysine par rapport au ritonavir est de 6:1 à 3:1.

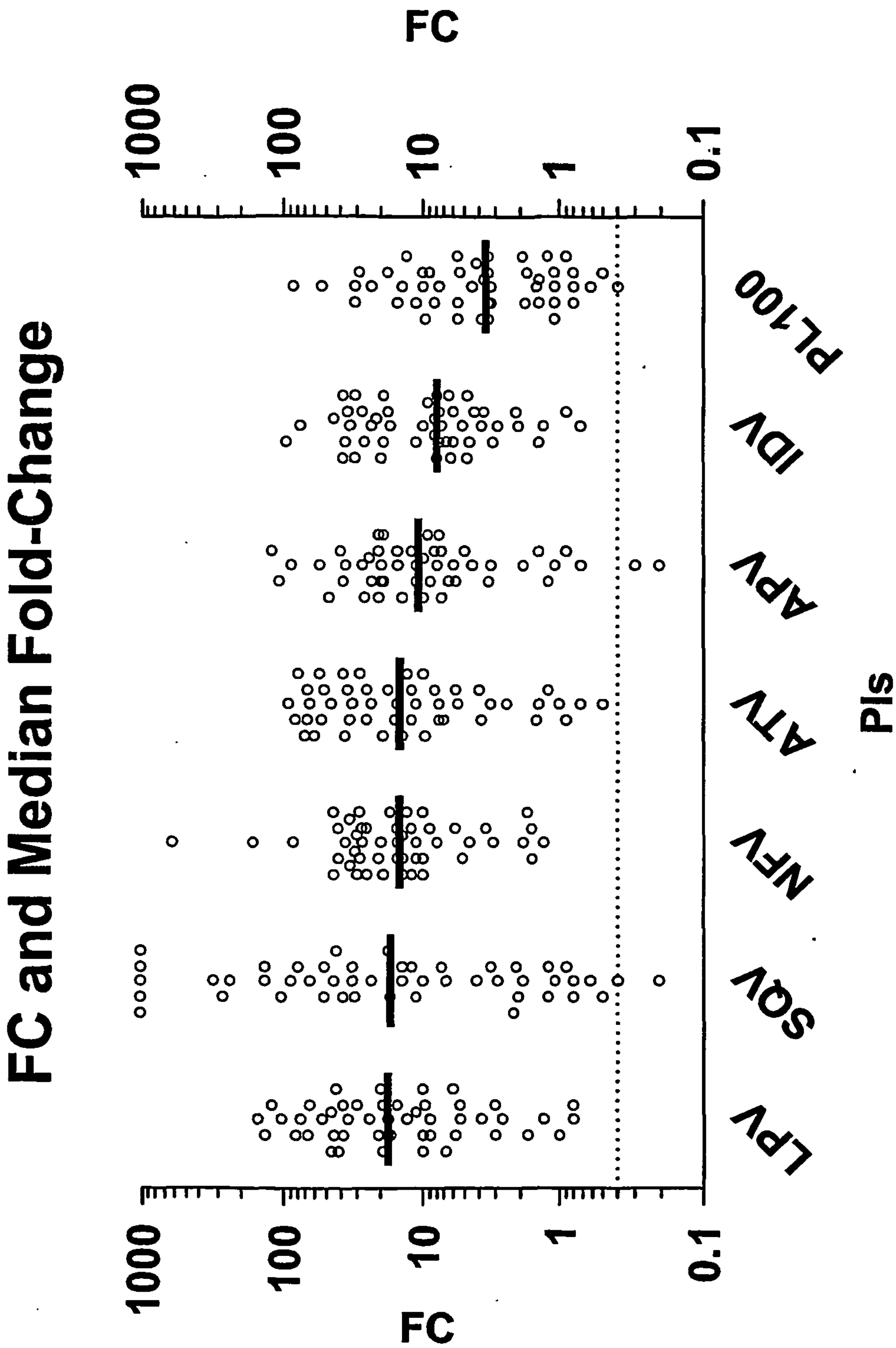
FC and Median Fold-Change
FC
1000
100
10
1
0.1
LPV
SQV
NFV
ATV
APV
IDV
PL100
PIs

Fig. 1

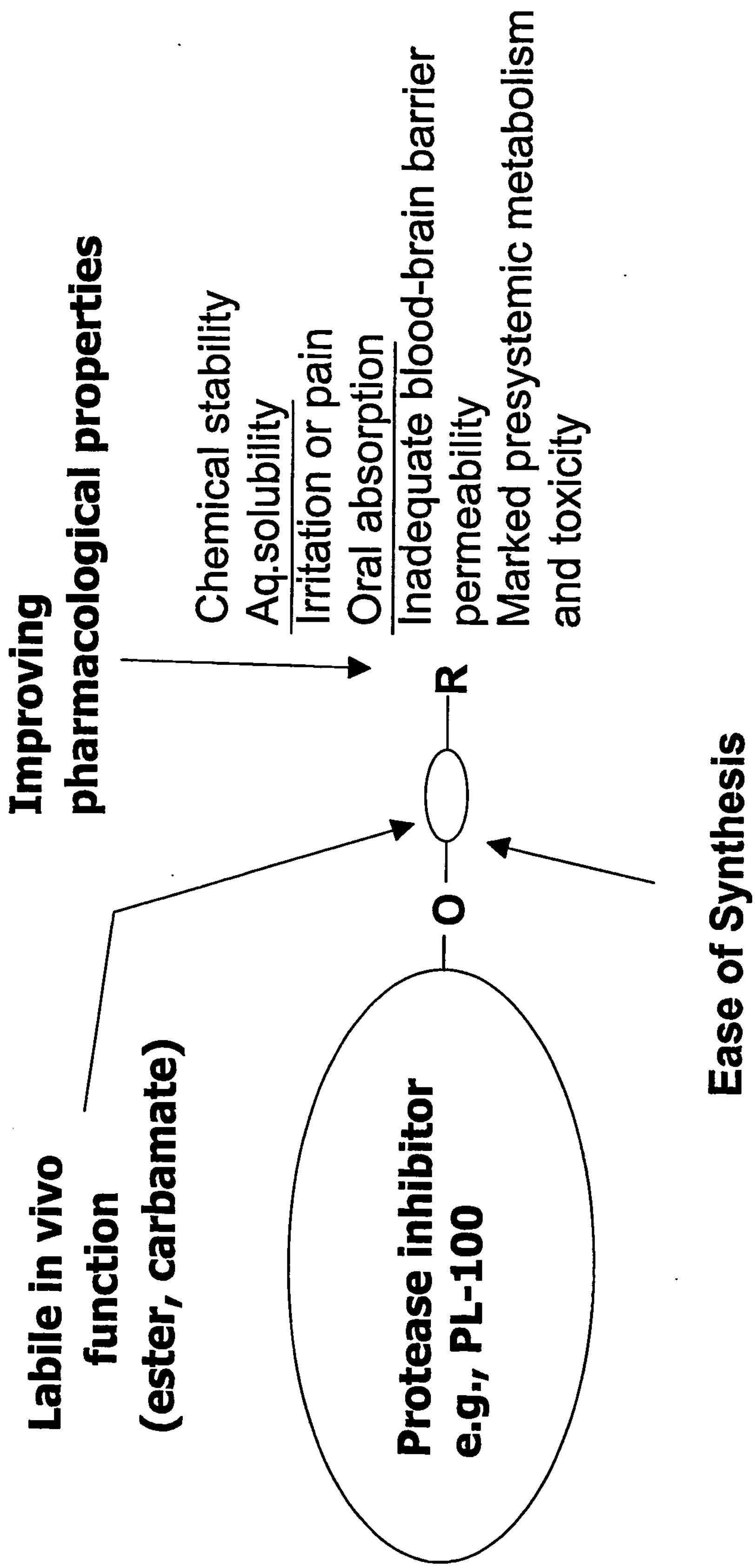
Labile in vivo
function
(ester, carbamate)
Improving
pharmacological properties
Chemical stability
Aq.solubility
Irritation or pain
Oral absorption
Inadequate blood-brain barrier
permeability
Marked presystemic metabolism
and toxicity
Protease inhibitor
e.g., PL-100
O
R
Ease of Synthesis

Fig-2

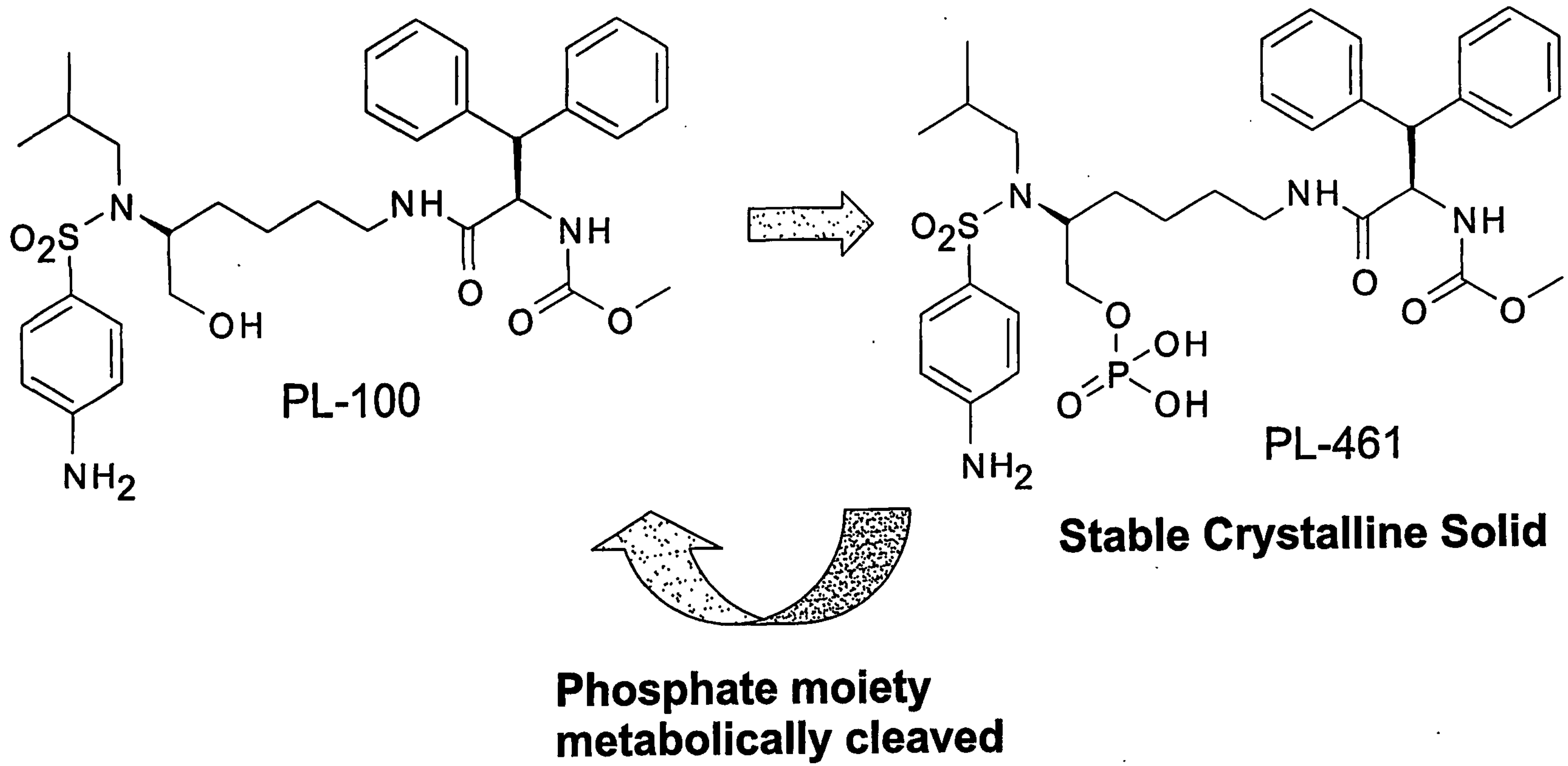


PL-100

PL-461

Fig. 3

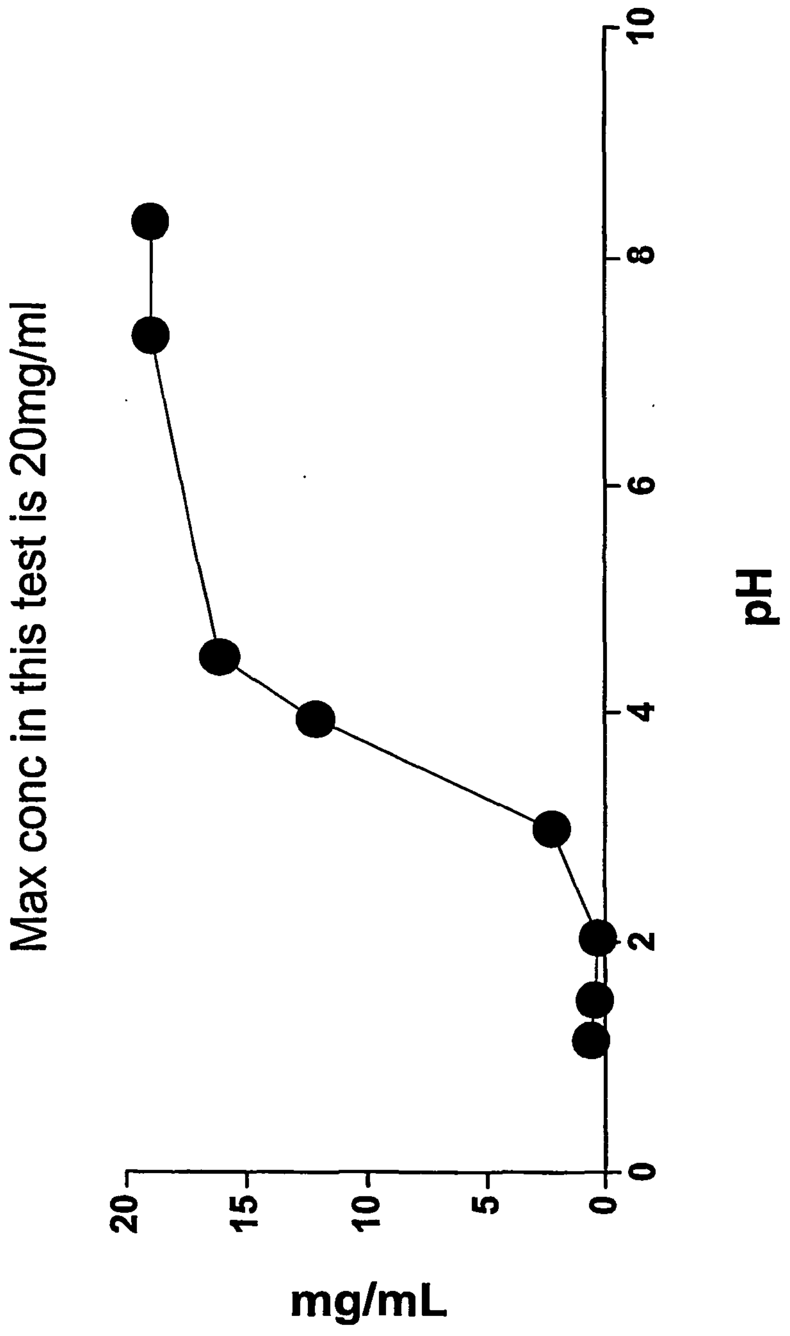


Fig. 4

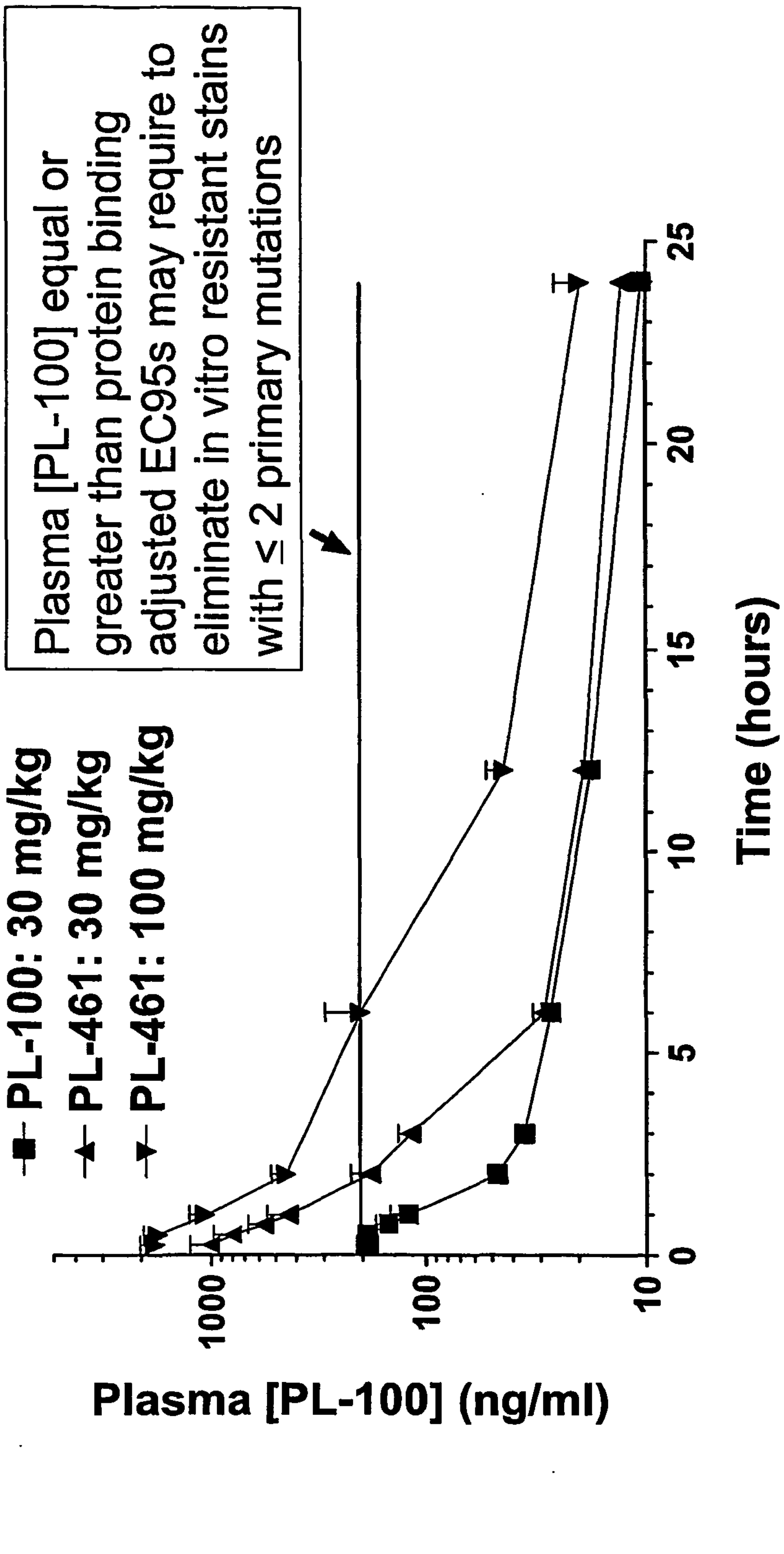

-■- PL-100: 30 mg/kg
-▲- PL-461: 30 mg/kg
-▼- PL-461: 100 mg/kg
Plasma [PL-100] equal or greater than protein binding adjusted EC95s may require to eliminate in vitro resistant stains with ≤ 2 primary mutations
Plasma [PL-100] (ng/ml)
1000
100
10
0
5
10
15
20
25
Time (hours)


Fig. 5

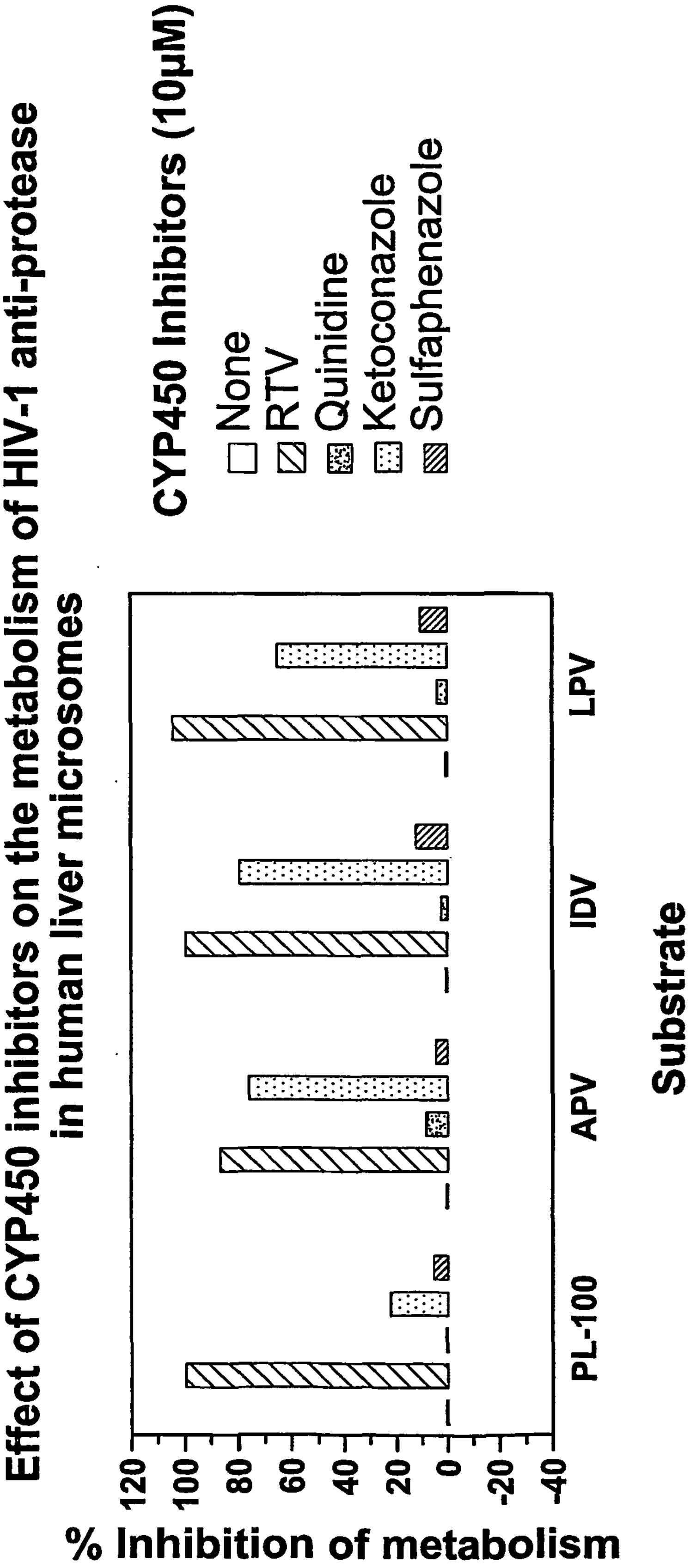
Effect of CYP450 inhibitors on the metabolism of HIV-1 anti-protease in human liver microsomes
CYP450 Inhibitors (10µM)
None
RTV
Quinidine
Ketoconazole
Sulfaphenazole
% Inhibition of metabolism
120
100
80
60
40
20
0
-20
-40
PL-100
APV
IDV
LPV
Substrate

Fig. 6

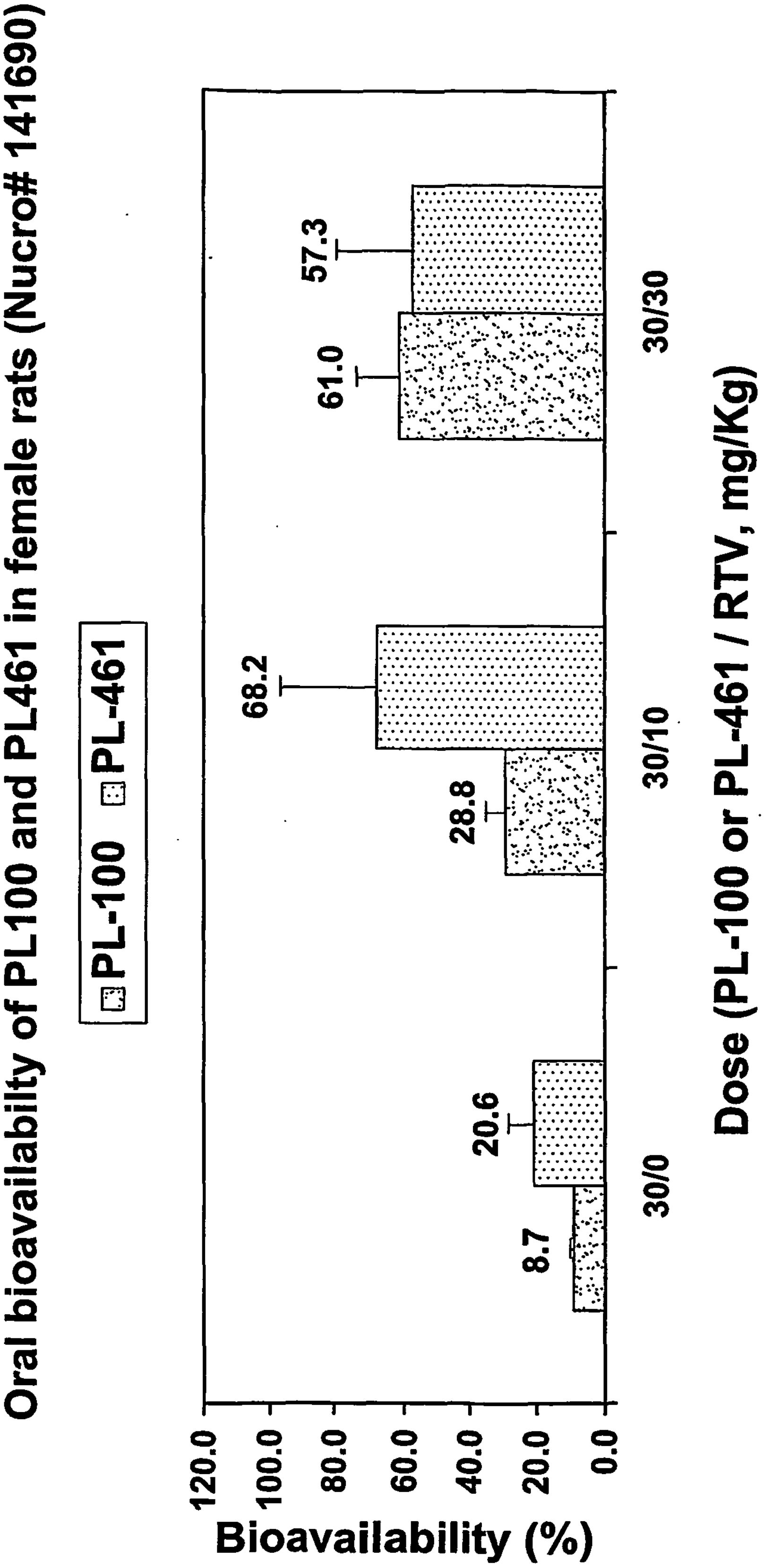
Oral bioavailabilty of PL100 and PL461 in female rats (Nucro# 141690)
PL-100
PL-461
Bioavailability (%)
120.0
100.0
80.0
60.0
40.0
20.0
0.0
8.7
20.6
28.8
68.2
61.0
57.3
30/0
30/10
30/30
Dose (PL-100 or PL-461 / RTV, mg/Kg)

Fig. 7

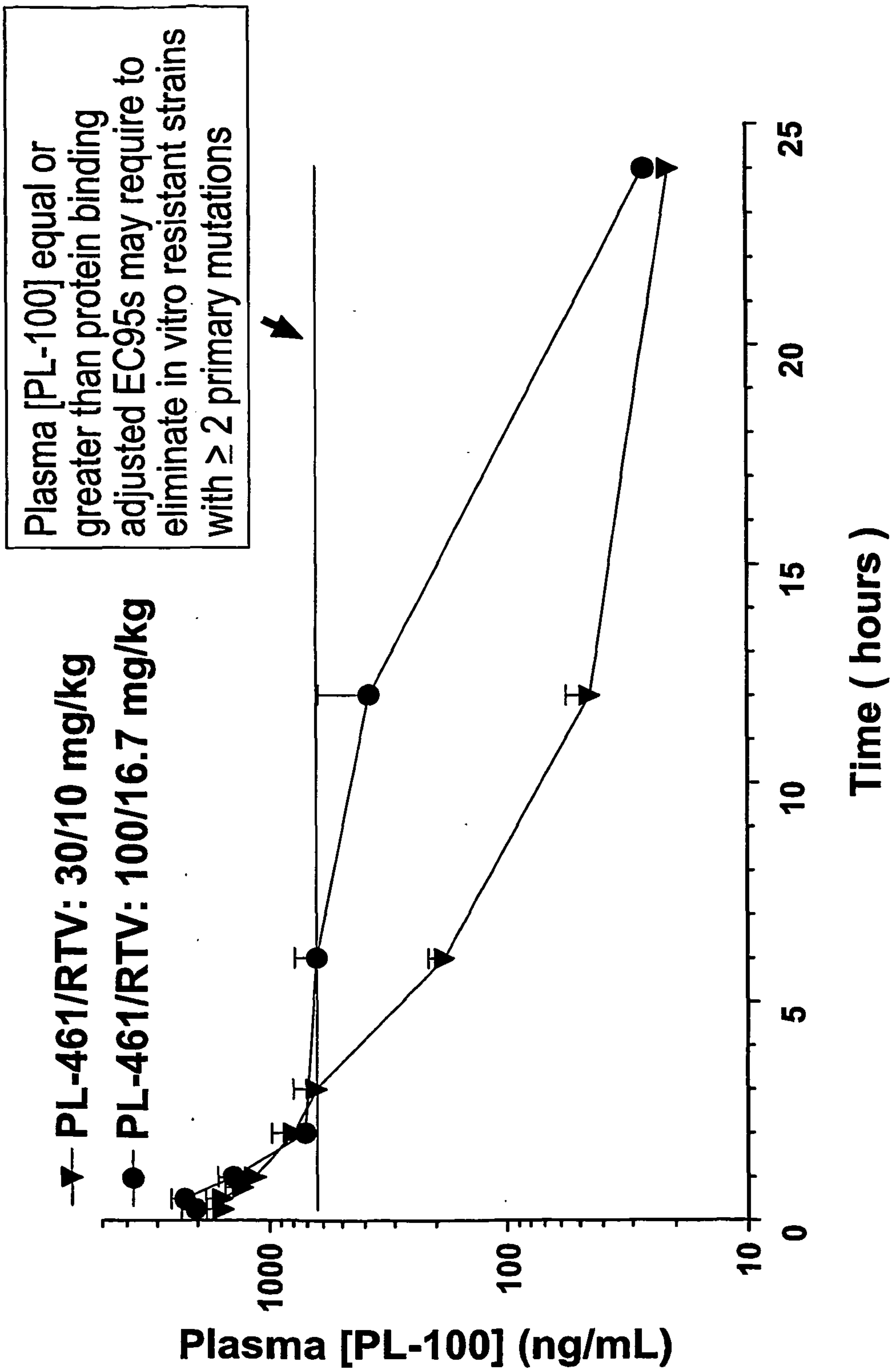

PL-461/RTV: 30/10 mg/kg
PL-461/RTV: 100/16.7 mg/kg
Plasma [PL-100] equal or greater than protein binding adjusted EC95s may require to eliminate in vitro resistant strains with ≥ 2 primary mutations
Plasma [PL-100] (ng/mL)
1000
100
10
0
5
10
15
20
25
Time ( hours )


Fig. 8

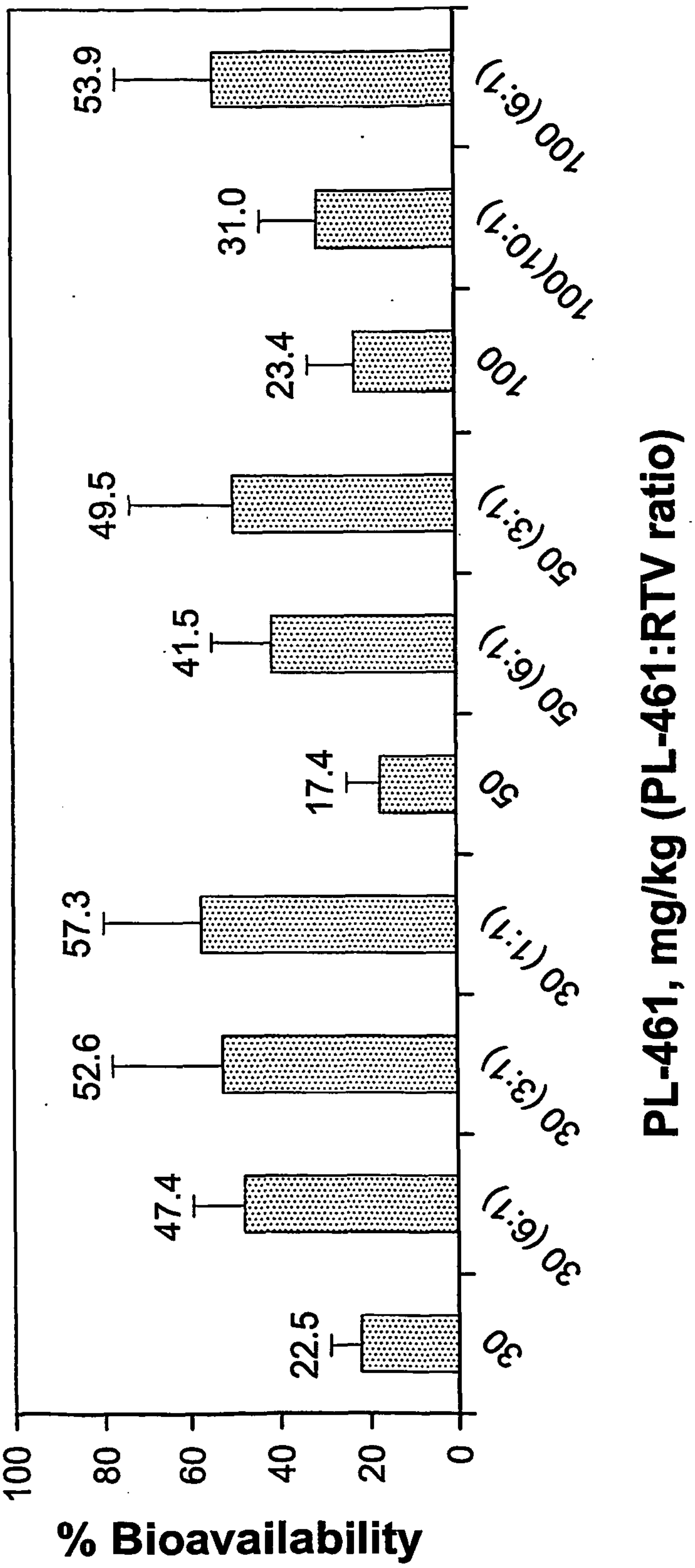
% Bioavailability
0
20
40
60
80
100
PL-461, mg/kg (PL-461:RTV ratio)
30
30 (6:1)
30 (3:1)
30 (1:1)
50
50 (6:1)
50 (3:1)
100
100(10:1)
100 (6:1)
22.5
47.4
52.6
57.3
17.4
41.5
49.5
23.4
31.0
53.9

Fig. 9

Effect of PL-100 and Ritonavir on selected Human CYP450s

| Enzyme | Probe Substrate | Metabolic Reaction | *K*i (μM) | |
|---|---|---|---|---|
| | | | PL-100 | Ritonavir |
| CYP2C9 | Tolbutamide | Tolbutamide Methy1-Hydroxylation | 13.3 ± 1.7 | 13.9 ± 2.0 |
| CYP2D6 | Dextromethorphan | Dextromethorphan *O*-Demethylation | 27.8 ± 2.3 | 2.7 ± 0.3 |
| CYP3A4/5 | Testosterone | Testosterone 6ß-Hydroxylation | 0.445 ± 0.050 | 0.033 ± 0.005 |

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6436989 B, Hale
- US 6632816 B, Stranix
- US 10902935 B
- US 20060025592 A1


### Non-patent literature cited in the description


- **T.W. GREENE ; P. G. M. WUTS.** Protective groups in Organic Synthesis. John Wiley & Sons, Inc, 1999
- **MEEK et al.** *Nature,* 1990, vol. 343, 90-92